# EUROPEAN PATENT SPECIFICATION

(11) **EP 2 296 674 B1**
(45) Date of publication and mention of the grant of the patent: **17.09.2014**
(21) Application number: 09761964.7
(22) Date of filing: 11.06.2009
(51) Int. Cl.: A61K 35/37, C12N 5/074

(54) **NOVEL ADULT PROGENITOR CELL**
NEUE ADULTE VORLÄUFERZELLE
NOUVELLE CELLULE PROGÉNITRICE ADULTE

(30) Priority: 13.06.2008 GB 0810841; 27.06.2008 GB 0811865; 31.10.2008 GB 0820012
(43) Date of publication of application: 23.03.2011
(73) Proprietor: University College Cardiff Consultants Limited, Cardiff CF24 0DE (GB)
(72) Inventor: STEPHENS, Philip, Cardiff CF14 4XY (GB); DAVIES, Lindsay, Catrina, Cardiff CF14 4XY (GB)
(74) Representative: Fyles, Julie Marie
(86) International application number: PCT/GB2009/001443
(87) International publication number: WO 2009/150415

(56) References cited:
- WO-A1-2008/132722
- MIMEAULT M ET AL: "Stem cells: A revolution in therapeutics - Recent advances in stem cell biology and their therapeutic applications in regenerative medicine and cancer therapies" CLINICAL PHARMACOLOGY & THERAPEUTICS, vol. 82, no. 3, September 2007 (2007-09), pages 252-264, XP009126807 ISSN: 0009-9236
- STEPHENS P ET AL: "Non-epithelial oral mucosal progenitor cell populations" ORAL DISEASES, STOCKTON PRESS, BASINGSTOKE, GB, vol. 13, no. 1, 1 January 2007 (2007-01-01), pages 1-10, XP002494289 ISSN: 1354-523X
- PHINNEY D G ET AL: "Concise review: Mesenchymal stem/multipotent stromal cells: The state of transdifferentiation and modes of tissue repair - Current views", STEM CELLS, ALPHAMED PRESS, DAYTON, OH, US, vol. 25, no. 11, 1 November 2007 (2007-11-01), pages 2896-2902, XP002612819, ISSN: 1066-5099, DOI: 10.1634/STEMCELLS.2007-0637 [retrieved on 2007-09-27]
- Keren Marynka-Kalmani ET AL: "The Lamina Propria of Adult Human Oral Mucosa Harbors a Novel Stem Cell Population", Stem Cells, vol. 28, no. 5, 1 May 2010 (2010-05-01), pages N/A-N/A, XP055044659, ISSN: 1066-5099, DOI: 10.1002/stem.425
- COLLINS ANNE T ET AL: "Identification and isolation of human prostate epithelial stem cells based on alpha2beta1-integrin expression", JOURNAL OF CELL SCIENCE, CAMBRIDGE UNIVERSITY PRESS, LONDON, GB, vol. 114, no. 21, 1 November 2001 (2001-11-01), pages 3865-3872, XP002354681, ISSN: 0021-9533
- B. Delorme ET AL: "Specific plasma membrane protein phenotype of culture-amplified and native human bone marrow mesenchymal stem cells", Blood, vol. 111, no. 5, 17 December 2007 (2007-12-17), pages 2631-2635, XP055119265, ISSN: 0006-4971, DOI: 10.1182/blood-2007-07-099622
- DOWTHWAITE GARY P ET AL: "The surface of articular cartilage contains a progenitor cell population", JOURNAL OF CELL SCIENCE, CAMBRIDGE UNIVERSITY PRESS, LONDON, GB, vol. 117, no. 6, 29 February 2004 (2004-02-29), pages 889-897, XP002478828, ISSN: 0021-9533, DOI: 10.1242/JCS.00912
- S Fickert: "Identification, quantification and isolation of mesenchymal progenitor cells from osteoarthritic synovium by fluorescence automated cell sorting", Osteoarthritis and Cartilage, vol. 11, no. 11, 1 November 2003 (2003-11-01), pages 790-800, XP055119269, ISSN: 1063-4584, DOI: 10.1016/S1063-4584(03)00167-5
- QUIRICI N ET AL: "Isolation of bone marrow mesenchymal stem cells by anti-nerve growth factor receptor antibodies", EXPERIMENTAL HEMATOLOGY, ELSEVIER INC, US, vol. 30, no. 7, 1 January 2002 (2002-01-01), pages 783-791, XP002973273, ISSN: 0301-472X, DOI: 10.1016/S0301-472X(02)00812-3
- JENSEN U B ET AL: "THE SPATIAL RELATIONSHIP BETWEEN STEM CELLS AND THEIR PROGENY IN THE BASAL LAYER OF HUMAN EPIDERMIS: A NEW VIEW BASED ON WHOLE-MOUNT LABELLING AND LINEAGE ANALYSIS", DEVELOPMENT, COMPANY OF BIOLOGISTS, CAMBRIDGE, GB, vol. 126, no. 11, 1 June 1999 (1999-06-01) , pages 2409-2418, XP008006076, ISSN: 0950-1991

## Description

The present invention relates to a novel progenitor cell(s) (PC) and its progeny; and a method of obtaining said PC. In addition, the invention relates to the use of said PC, for the production of replacement tissues for individuals suffering from, for example, facial burns or injuries; and to the provision of neuronal cells with the potential for neuronal regeneration. The invention also relates to the use of said PC for the treatment of immune-related disorders including, but not limited to, graft-versus-host disease (GVHD) and chronic conditions such as diabetes.

### Introduction

The isolation and use of adult stem cells (ASCs) for tissue engineering applications has received great interest due to the ethical and moral issues surrounding the use of pluripotent embryonic stem cells (ESCs). ASCs have been isolated from multiple sites within the body such as the skin, periosteum, dental pulp, umbilical cord, tendon and periodontal ligament. However, bone marrow stromal cells (BMSCs) are the most characterised. The quest to find alternative ASC sources to that of the bone marrow is perpetuated by the decreasing numbers of BMSCs with age and their significant loss in proliferative and differentiation capacity(1). ASCs are self-renewing and multipotent which lends them to therapeutic application. Their limited lifespan *in vitro* also acts as an advantage over ESCs by lowering their potential tumourogenicity. The tissue that these cells are isolated from determines their potential for therapeutic application, with some ASC sources being solely able to differentiate into tissues from which they originate, for example cortex isolated progenitor cells(2).

It is therefore important to take into consideration the range of lineages an ASC can generate upon induction and their proliferative capacity in the search for the ideal biopsy site for tissue engineering purposes. Indeed, the isolation of sufficient numbers of stem cells from adult tissues can be extremely limiting. Some ASCs have been characterised as having no/low active telomerase, making their *in vitro* expansion difficult and restricted (2,3).

Furthermore, the isolation of ASCs is often invasive, for example the need for bone marrow aspiration for the isolation of BMSCs or a skin biopsy for the isolation of skin derived precursors; the latter resulting in scar formation.

Wound healing within the oral mucosa is characterised by reduced inflammation, rapid re-epithelialisation and differential fibroblast responses resulting in minimal scar formation; a process generally considered to be more 'foetal-like' in comparison to that observed within the skin (4-8).
Progenitor cells (PCs) have been reported to exist within the basal layer of the oral mucosa epithelium (9-11), but to date there are no reports of the existence of a progenitor or stem cell population resident within the oral mucosa lamina propria (OMLP). A recent review has highlighted the potential for the existence of a progenitor or stem cell population within the OMLP through reference to PC populations within the dermis of the skin (12). Traditionally, PC's are described as unipotent or multipotent cells. From this point of view, they may be compared to adult stem cells. But progenitors are said to be in a farther stage of cell differentiation. They are 'in between' stem cells and fully differentiated cells. The kind of potency they have depends on the type of their "parent" stem cell and also on their niche. Like stem cells, mostly, they are formed in a colony, with the right conditions for them to grow and differentiate into their target tissues. Lots of properties are shared by progenitor cells and adult stem cells, however, both sets of cells are different from Embryonic stem cells (ESCs) which are true stem cells in that ESCs are pluripotent and show unlimited capacity for self-renewal. In contrast, many cells termed adult stem cells would be better defined as progenitor cells, as their capacities for unlimited self renewal and plasticity have not been comprehensively demonstrated. Progenitor cells act as a repair system for the body. They replenish specialised cells, but also maintain the blood, skin and intestinal tissue.

We have previously reported that patient matched oral mucosal and skin fibroblasts have distinct phenotypic and genotypic differences which result in the preferential healing response seen within the oral mucosa irrespective of wound environment (6-8) (Enoch et al., 2009 in press). These findings support the idea that a PC population may be resident specifically within the OMLP and it is this cell population that may contribute to the preferential healing response seen on wounding of this tissue. Importantly, isolation of a PC population from the OMLP would offer distinct advantages for therapeutic applications, providing a site of biopsy that is easily accessible and minimally invasive, with rapid healing and no/minimal resultant scar formation for the patient.

We disclose herein for the first time the existence of a novel PC population resident within the lamina propria of the oral mucosa. This cell population can be reliably isolated from buccal mucosa biopsies and we have, surprisingly, discovered that this PC population has great potential for therapeutic application because it can differentiate into a range of lineages and, moreover, this PC population has advantageous proliferative capacity enabling a sufficient number of cells to be produced for therapeutic purposes. Indeed, clones originating from a single OMLP PC can be rapidly expanded *in vitro,* aided by the surprising expression of active telomerase. It is these clonal populations that are multipotent and can be differentiated into cell lineages of both mesenchymal and neuronal origin.

We also disclose herein for the first time the finding that our novel PC population has immunomodulatory, more specifically immuno-suppressive, potential. Indeed, we have found that the co-culture of only a few of our PC with lymphocytes can suppress an immune response. This has important implications for the allogeneic transfer of cells or tissue as the use/presence of our PC should safeguard against tissue rejection.

To place this into context, Mesenchymal stem cells (MSCs) have previously been shown to exert an immunomodulatory effect on the adaptive immune system. Recent work published using human periodontal ligament stem cells (also of mesenchymal origin) have demonstrated that direct contact with lymphocytes generates the most efficient immunosuppressive effect (13). The degree of suppression seen within MSCs is dose-dependent, with decreasing levels of immunosuppression seen with decreasing numbers of MSCs present. In contrast our OMLP PCs act in a contact-independent and dose-independent manner to produce suppression of an allogeneically induced immune response. This suppressive effect may be an extremely important feature of our OMLP PCs because mortality due directly to severe Graft-Versus-Host-Disease (GVHD) remains one of the major obstacles in allogeneic haematopoietic stem cell (HSC) transplantation. GVHD is characterised by a direct attack by T-cells present within the HSC graft on the 'antigenically foreign' host. Limited clinical trials have been reported in the treatment of GVHD. A recent study by Ringden et al. demonstrated that in 8 patients infused with MSCs, 6 of those patients showed a complete response, with removal of all symptoms (14). It therefore follows that our progenitor cells, or their progeny, may be used as a therapeutic, *per se,* for modulating an immune response and so treating inflammatory disorders such as GVHD in allogeneic haematopoietic stem cell (HSC) transplantation.

Moreover, it also follows that our progenitor cells, or their progeny, may be used as a therapeutic, *per se,* for modulating an immune response and so treating immune disorders such as, but not limited to, GVHD.

We have therefore discovered that the OMLP offers a preferential cell source for tissue engineering applications by providing a self-renewing and multipotent PC population from a minimally invasive biopsy site which heals rapidly and with minimal scar formation. We have further discovered that this OMLP PC population has advantageous immunomodulatory activity when compared to other PCs or ASCs.

### Statements of Invention

An isolated human adult progenitor cell (PC), wherein said cell is derived from the lamina propria of the oral mucosa and further wherein said cell is multipotent, self-renewing and immunomodulatory and expresses each one of the following markers characteristic of said progenitor cells: hTERT, Nanog, Oct4, Sox2, KLF-4, Sox10, Slug, Snail and Twist.

Reference herein to multipotent concerns the ability to differentiate into a number of cell types as described herein.

Reference herein to self-renewing includes reference to renew but not in an unlimited sense.

Reference herein to immunomodulatory concerns the ability of our PC, or their progeny, including differentiated cells, to suppress an immune response in either a contact-independent or dose-independent manner or independently of induction of HLA II cell surface expression.

In a preferred embodiment of the invention said PC is positive for the stem cell marker triplicate CD90, CD105, & CD166.

Further, ideally, said PC is negative for the cell markers CD34 and CD45.

Yet further still, said PC is positive for any one or more of the following cell markers CD90, CD105, CD166, STRO-1, CD44, CD146, Notch1/2/or 3, Delta 1 or Jagged 2.

Reference herein to a cell being positive or negative for a specified cell marker concerns the ability of said cell to express said marker wherein positive signifies expression of said marker and negative signifies lack of expression of said marker.

As taught herein said PC undergoes in excess of 45 population doublings and, ideally, also expresses telomerase either throughout this doubling time or during a part thereof.

As mentioned, in preferred embodiments of our invention said PC is positive for the transcription factor Nanog at the mRNA level (although not necessarily at the protein level), which is thought to be key in maintaining pluripotency in embryonic stem cells. Furthermore, said PC is also positive for the transcription factor Oct4, which is involved in undifferentiated self-renewal of stem cells, it is expressed within cells freshly isolated after digestion of the oral mucosal lamina propria with collagenase (however, it is lost after culture in traditional mesenchymal stem cell medium). In addition, said PC is positive for Sox2 a transcription factor important in regulating the self-renewal of undifferentiated embryonic stem cells and controlling Oct4 expression and KLF-4 another transcription factor which appears to be important in the maintenance of stem cells. Our PC is also positive for the neural crest markers Sox10 (which is important for neural crest and peripheral nervous system development); Slug, Snail and Twist as well as being positive for the developmental marker Notch 1.

The presence of these markers has implications for the target cell types into which said PC can be differentiated. Indeed the expression of the above embryonic markers, at either the mRNA or protein level, and immature developmental markers within the progenitor cells indicates that there may be a preferential cell type, requiring less modification than the cell types currently used, for the future development and utilisation of induced pluripotent stem cell (iPS) technology. Indeed, in this respect it is of note that our PC express the main four cell factors (Nanog, KLF-4, Sox2, and Oct4) which are used in iPS to reprogramme fibroblasts back to embryonic-like stem cells. That these four factors are naturally expressed within the PC of OMLP suggests we have a cell population which can be isolated and acts like embryonic stem cells without requiring iPS reprogramming. Thus we have a PC population with pluripotent ability and/ or further we have a PC population which requires less reprogramming than other cell types to produce an embryonic stem cell type cell.

Therefore, it is taught herein that there is provided an iPS comprising a PC of the OMLP that expresses, or is positive for, the four following cell markers: Nanog, KLF-4, Sox2, and Oct4.

Additionally, in a further aspect of the invention there is provided a method for obtaining progenitor cells from the lamina propria of the oral mucosa by a method comprising the steps of:
a) treating a sample from the oral mucosa of a subject by removal of fatty tissue and the epithelial layer of the cells;
b) removing the remaining lamina propria extracellular matrix to generate a mixed suspension of cells;
c) isolating potential progenitor cells and allowing them to form colonies; and
d) selecting colonies with each one of the following markers characteristic of progenitor cells: hTERT, Nanog, Oct 4, Sox2, KLF-4, Sox10, Slug, Snail and Twist.

In step (a), the fatty tissue may be removed by any convenient means, for example by dissecting a biopsy sample. Pronase/dispase are suitable enzymes for removing the epithelial layer of the oral mucosa. Treatment such as 2mg/ml, overnight at 37ºC will suffice, although those skilled in the art may use variations on this theme.

In step (b), the lamina propria extracellular matrix may be removed by digestion with collagenase. Treatment such as overnight incubation at 37ºC in 1mg/ml *Clostridium histolyticum* A collagenase will suffice, although those skilled in the art may use variations on this theme.

In step (c) above potential progenitor cells can be isolated by differential adhesion to a cell binding agent such as fibronectin or a fragment containing an RGD sequence of amino acids as reported by Dowthwaite *et al,* 2004 and Jones and Watt, 1993 (10,15). Single cells adhered to the fibronectin substrate may then be allowed to form colonies (>32 cells) over 12-14 days in culture and the resulting colonies may be isolated by trypsinisation within a cloning ring. Alternatively, other substrates such as such as collagen and laminin can be used as cell binding agents for differential adhesion in step (c) above as discussed in the Jones and Watt paper. Moreover, if sufficient cells are isolated, cells can be sorted using flow cytometry or Magnetic-Activated Cell Sorting (MACS) selection.

We have specifically used a clonal population of cells (rather than heterogenous isolations from unsorted whole tissue) for this study as this allows accurate definition of a pure cell population. It is also taught herein that heterogenous isolation from unsorted whole tissue may be employed using conventional techniques. These techniques may include, for example, the isolation of potential progenitor cells by differential adhesion to, for example, a cell binding agent such as fibronectin, an RGD sequence of amino acids, collagen or laminin as reported by Dowthwaite et al (2004) and Jones and Watt (1993) (10,15). Single cells adhered to, for example, fibronectin substrate can be allowed to grow to confluence before being passaged and expanded as a heterogeneous progenitor cell culture in monolayer culture.

In step (d) above, markers characteristic of progenitor cells include telomerase which is thought to be key in maintaining self renewal in embryonic stem cells; and the transcription factor Nanog which is thought to be key in maintaining pluripotency; and the transcription factor Oct4 which is involved in undifferentiated self-renewal of stem cells. Further markers assayed for are KLF4 and neural crest markers, including Sox10 (which is important for neural crest and peripheral nervous system development); Sox2, Slug, Snail, Twist.. Another useful marker that may be assayed is the developmental marker Notch1. Other markers which may be assayed for include CD90, CD105, CD166, STRO-1, CD44, CD146, CD34, CD45,, p75, , Notch 2 or 3, Delta 1 and Jagged 2. With the exception of CD34 and CD45, one would be looking for the selected cells to be positive for any one or more of the aforementioned markers and preferably positive for more than one, for example, in increasing order of preference, at least two, three, four, five or six or more of these markers. To confirm these progenitor cells are not of a haematopoietic origin one would be looking for the selected cells to be CD34 and CD45 negative.

The presence or absence of the markers may be determined by standard methods, preferably immunocytochemical methods or Fluorescence-activated cell sorting (FACS) analysis.

Accordingly, in a further preferred method of the invention said method comprises a further step that involves, after step (c) and before performing (d), determining the expression of any one or more of the above specified markers using conventional methods such as, but not limited to, immunocytochemical methods or Fluorescence-activated cell sorting (FACS) analysis.

It is taught herein that the method may further include the additional step of:
e) expanding the colonies selected in step (d).

Usually, these selected colonies will be expanded in monolayer culture and this may continue until senescence.

As will be known by those with experience, differentiation of progenitor cells to a selected phenotype can be achieved by changing the culture conditions. For example, the inventors have found that cells cultured in Dulbecco modified Eagle's medium (DMEM) with 10% foetal calf serum demonstrate a fibroblast-type morphology and maintain expression of the stem cell marker triplicate CD90, CD105 and CD166 and ideally also the series STRO-1, CD44, CD146, moreover, they are also ideally CD34 and CD45 negative. However, by varying the culture medium, it is possible to obtain cartilage cells, muscle cells, adipocytes, bone cells and neuronal cells. Further details of culture media are set out in the examples below.

However, briefly, it is disclosed that cartilage cells were obtained by culturing PCs in a pellet culture system in basal media (Dulbecco's modified Eagles medium [DMEM] supplemented with 2mM L-glutamine, 100U/ml penicillin G, 100µg/ml streptomycin sulphate, 0.25µg/ml amphotericin B) supplemented with 1 x insulin, transferrin and selenium (ITS) supplement (Invitrogen, UK), 50µg/ml L-ascorbate (Sigma, UK) and 5ng/ml transforming growth factor beta 1 (TGFβ1) (Peprotech EC Ltd., UK).

Bone cells were obtained using osteogenic induction media (Alpha modified Eagles medium (α-MEM) (Invitrogen, UK) supplemented with 10% (v/v) FCS, 2mM L-glutamine, 100U/ml penicillin G, 100µg/ml streptomycin sulphate, 0.25µg/ml amphotericin B, 50µg/ml L-ascorbate, 10µM dexamethasone and 5mM beta-glycerophosphate (Sigma, UK)).

Adipocytes were obtained using adipogenic induction media (SCM supplemented with 10µg/ml insulin, 1µM dexamethasone, 100µM indomethacin, 100µM 3-isobutyl-1-methyl-xanthine [IBMX] (Sigma, UK)) for 3 days and then switched to adipogenic maintenance media consisting of SCM supplemented with 10µg/ml insulin for a further 24 hours. Cells were then refed with induction media. This cycle of media was repeated for 28 days.

PC's for Neuronal and Schwann cells lineage were grown using X-VIVO™10 medium (Lonza Group Ltd., UK) supplemented with 0.1mM β-mercaptoethanol (Sigma, UK), 1% (v/v) non-essential amino acids (Invitrogen, UK), 100U/ml penicillin G, 100µg/ml streptomycin sulphate, 0.25µg/ml amphotericin B and 80ng/ml recombinant human basic fibroblast growth factor (rhbFGF) (Peprotech EC Ltd., UK).

These cells were subjected to neuronal differentiation using previously described protocols (16,17). Briefly, single cell suspensions were counted and seeded onto 0.1% Matrigel™ (BD Biosciences, UK) coated chamberslides at 5.6x10³ cells/cm² in DMEM-F12 (3:1) media supplemented with 40ng/ml hrbFGF, 10% (v/v) FCS, 2mM L-Glutamine, 100U/ml penicillin G, 100µg/ml streptomycin sulphate and 0.25µg/ml amphotericin B. Cells were cultured for 7 days with media changes every 3 days. After 7 days in culture, the rhbFGF was removed from the culture media and replaced with 10ng/ml nerve growth factor (NGF), 10ng/ml brain derived growth factor (BDNF) and 10ng/ml neurotrophin 3 (NT3). Cells were cultured for an additional 7 days with media changes every 3 days.

Schwann cell differentiation was as previously described by Toma et al., (2005) (18). Collagenase treated cells were seeded onto poly-D-lysine laminin coated chamberslides (2µg/ml) at an equivalent density to that described for neuronal differentiation. Cells were cultured in DMEM-F12 (3:1) with 10% (v/v) FCS, 2mM L-Glutamine, 100U/ml penicillin G, 100µg/ml streptomycin sulphate and 0.25µg/ml amphotericin B for 7 days. Media was then replaced with neurobasal media (Invitrogen, UK) containing 1% (v/v) FCS, 1% (v/v) N2 supplement (Invitrogen, UK), 4µM forskolin (Sigma, UK), 2mM L-Glutamine, 100U/ml penicillin G, 100µg/ml streptomycin sulphate, 0.25µg/ml amphotericin B and 10ng/ml heregulin β1 (Peprotech EC Ltd., UK). Cells were maintained in this media for a further 7 days. Media was changed every 3 days, with 50% of the conditioned media being reapplied to the cells.

Accordingly, it is taught herein that the above method further includes a last step involving the differentiation of the said selected PC wherein differentiation is effected by changing the culture conditions of said PC in a selected way, as herein described, to produce a selected differentiated cell.

Additionally, it is disclosed that there is a method for obtaining muscle cells, adipocytes, chondrocytes, bone cells, Schwann cells and/or neuronal cells, the method comprising differentiating at least one progenitor cell (PC) obtained from the lamina propria of the oral mucosa, or its progeny.

In the context of the present invention, it is taught that "muscle cells" refers to both smooth and striated muscle cells and "bone cells" refers to osteoblasts and osteocytes.

It is taught that there is provided at least one differentiated cell obtained by differentiating at least one progenitor cell isolated from the lamina propria of the oral mucosa.

Ideally, it is taught that said differentiated cell is a cartilage cell or a muscle cell or an adipocyte or a bone cell or a Schwann cell and/or a neuronal cell.

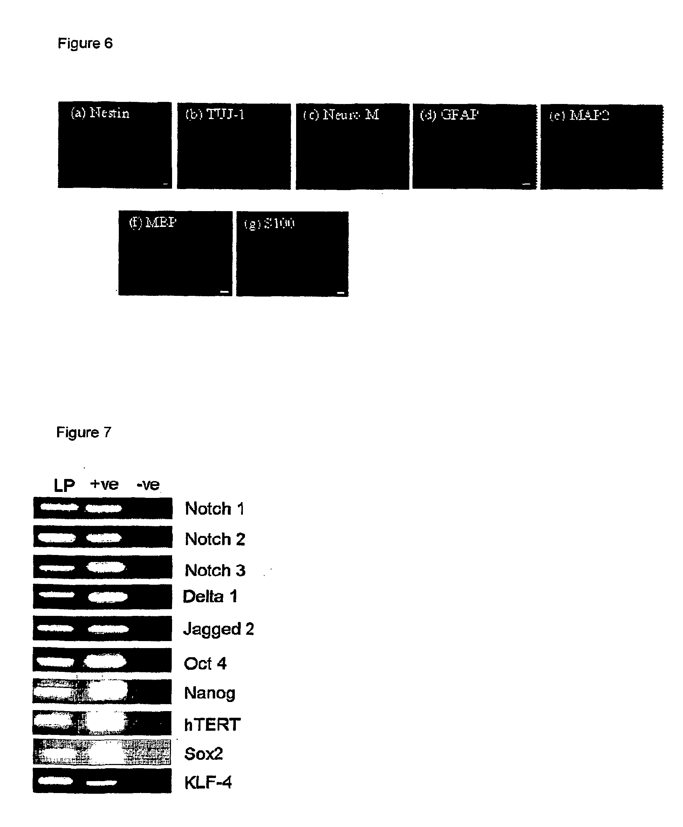

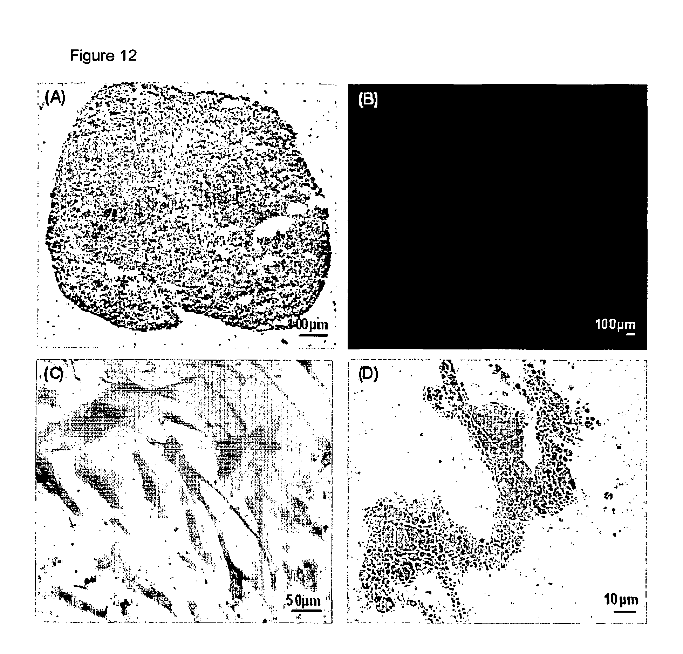

Examples of such differentiated cells include cells able to produce cartilage (confirmed by the markers aggrecan+, Sox9+, col2a1 type A+), adipocytes (confirmed by expression of oil red O staining and, ideally, also expression of lipoprotein lipase, CAATT/Enhancer binding protein alpha [CEBPα] and peroxisome proliferator-activated receptor gamma [PPARγ] at the messenger RNA level), bone cells (confirmed by Von Kossa and Alkaline Phosphatase staining) (Fig 12), Schwann cells (confirmed by S100 and myelin basic protein (MBP) production by immunocytochemistry) and neuronal cells (confirmed by production of nestin, beta III tubulin (TUJ-1), neurofilament M, glial fibrillary acidic protein (GFAP) and microtubule associated protein 2 (MAP2) by immunocytochemistry; also see Figure 6a-g).

It is taught herein that said differentiated cell is obtained using the method described above and so is a clonally derived cell.

Further, it is taught herein that one can obtain re-engineered tissue by culturing the said differentiated cell.

The said PC and, as taught herein the re-engineered tissue, may be of use in various medical applications and therefore it is taught that a differentiated cell or re-engineered tissue as disclosed herein is of use in medicine.

For example, it is taught herein that bone cells, muscle cells, cartilage, adipocytes and re-engineered tissues formed from these cells may all be used in the treatment of wounds or burns or repairing damage caused by disease, for example cancer. This is particularly significant for the treatment of facial injuries and scarring.

Further, it is taught that Neuronal cells may be used for neurological disorders such as Parkinson's. Schwann cells could be used for nerve damage repair and in the treatment of spinal cord injury.

Advantageously, said PC(s) or their progeny, including the differentiated cells, may be derived from the patient to be treated but, alternatively, may be derived from another individual and used in allogeneic tissue engineering and transplantation. This latter option is available to those working the invention because of the inherent immunomodulatory nature of said PC, or their said progeny, and, in particular, the ability of these cells to suppress an immune response.

Further, said PC, or their said progeny, derived from an individual to be treated or any another person, may be co-administered with differentiated tissue derived from an individual other than the individual to be treated and in this instance said PC, or their progeny, exerts a beneficial immunosuppresive effect.

Therefore, there is taught herein a method for the treatment of wounds, burns, or repairing damage caused by disease, the method comprising replacing damaged tissue with differentiated cells or re-engineered tissue as described above wherein said differentiated cells or said tissue are either derived from the patient to be treated or derived from PC of the OMLP of another individual.

Further, there is taught herein a method for the treatment of wounds, burns, or repairing damage caused by disease, the method comprising coadministering i) differentiated cells or tissue derived from an individual other than the individual to be treated, which cells or tissue are not derived from PC of the OMLP, with ii) PC, or their progeny, of the OMLP.

Due to the immuno-suppressive nature of PC, or their progeny, of the OMLP said PC in part ii) above may be derived from any individual. An example of this latter aspect of the invention is the co-transfusion of haematopoietic stem cells from an individual, other than the individual to be treated, and our PC to safeguard against, or for the treatment of, GVHD.

Additionally, it is taught herein a method for the treatment of an inflammatory disorder or immune disorder comprising administering to an individual to be treated progenitor cells, or their progeny including differentiated cells or tissue, as described above or progenitor cells, or their progeny, as obtained by the method described above

In yet a further aspect of the invention there is provided use of the progenitor cells, or their progeny, as described above, or obtained according to the method described above, as a medicament.

In a preferred embodiment said use is to treat any one or more of the following disorders: wounds, burns, tissue damage, inflammation, autoimmunity, GVHD allogeneic haematopoietic stem cell (HSC) transplantation, diabetes or an immune disorder.

The invention will now be described in greater detail with reference to the figures in which:
**Figure 1** shows p75 positive cells coated onto fibronectin and isolated by differential adhesion.
**Figure 2** shows the differing morphology in colonies expanded in (a) DMEM/10% FCS, (b-c) X-VIVO10 media. (b) demonstrates a typical neurosphere like structure attached to the laminin substrate and (c) demonstrates cell migrating out of such a sphere and adopting a neuronal like morphology in contrast to the fibroblastic morphology seen in (a).
**Figure 3** shows the typical morphology of SY5Y neuroblastoma cell line. Single differentiated neuronal cells are seen migrating out of clusters of undifferentiated cells attached to the culture plate (http://en.wikipedia.org/wiki/SHSY5Y).
**Figure 4** Photomicrograph of a neurosphere generated when isolated colonies are expanded in monolayer culture using X-VIVO10 media conditions as described by Li et al., 2005. Bar=50µm
**Figure 5** Immunolocalisation of ***neuronal markers*** (a) Neurofilament M (-ve), (b) TUJ-1 (+ve) and (c) GFAP (+ve) and ***Schwann cell markers*** (d) MBP (-ve) and (e) S100 (+ve) in undifferentiated OMLP progenitor cells cultured in X-VIVO10 media by immunocytochemistry with a FITC conjugated secondary antibody. Bar=50µm. MAP2 data is not shown as no signal was seen.
**Figure 6** Immunolocalisation of ***neuronal markers*** (a) Nestin, (b) TUJ-1 (c) Neurofilament M, (d) GFAP and (e) MAP2 and ***Schwann cell markers*** (f) MBP and (g) S100 in differentiated OMLP progenitor cells by immunocytochemistry with a FITC conjugated secondary antibody (all +ve). Bar=10µm (50µm in Fig. 6e)
**Figure 7** **shows the Positive Expression of Embryonic and Developmental Markers within the OMLP.** Positive expression of embryonic (Nanog, Oct4, Sox2, KLF-4 and hTERT) and developmental markers (Notch 1, 2, 3, Delta 1 and Jagged 2) within whole OMLP digests was determined by RT-PCR. HuES9 ESC RNA was used as a positive control for all reactions.
**Figure 8** **shows PCs can be Reliably Isolated from the OMLP and Rapidly Expand *In Vitro***
   PCs were clonally expanded from OMLP digests by differential adhesion (A). Multiple PCs clones were isolated from three patients, which demonstrated a fibroblast-like morphology (A, B) and rapidly proliferated in vitro, totalling >50 PDs before reaching cellular senescence (C, D, E).
**Figure 9** **shows OMLP PCs Express Stem Cell Markers and are not of Haematopoietic or Fibrocyte Origin**
   FACS analysis was performed on expanded clones for the expression of the stem cell markers CD90, CD105, CD166 and CD44, and the haematopoietic/fibrocyte markers CD34 and CD45. PC clones isolated from 3 patients were confirmed as CD90⁺, CD105⁺, CD166⁺, CD44⁺, CD34⁻ and CD45⁻.
**Figure 10** **shows OMLP PCs Express Active Telomerase**
   PC colonies demonstrated expression of telomerase by ICC (A). Expression was quantified after monolayer expansion by Q-TRAP. All clones (n=3) were positive for active telomerase (B, C, D). Heat inactivation (HI) was used as a control. Separation of Q-TRAP products on acrylamide gels confirmed the telomeric repeat 'step-ladder' effect (E).

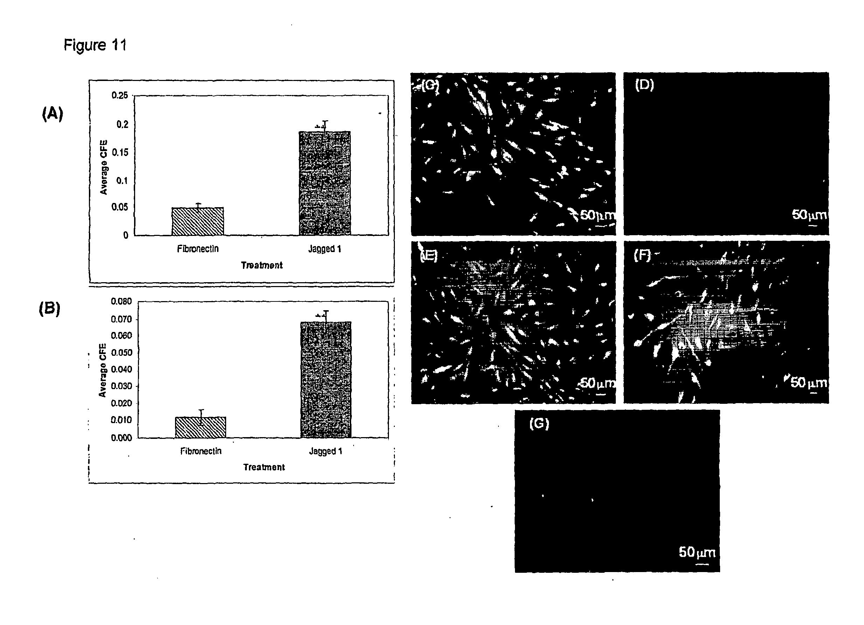

**Figure 11** **shows OMLP PCs Originate from the Neural Crest**
   CFE of PCs from two patients was significantly higher in the presence of Jagged 1 (A, B, C). A neural crest origin was confirmed by expression of the neural crest markers (D) Snail, (E) Slug, (F) Sox10, (G) Twist and (H) Notch 1 in developing colonies by ICC.
**Figure 12** **shows OMLP PCs can Differentiate into Mesenchymal Cell Lineages**
   PCs were differentiated down mesenchymal cell lineages. Differentiation down the chondrogenic lineage was confirmed by Van Gieson's staining (A) and aggrecan ICC (B). Osteoblastic induction was analysed by Von Kossa staining for calcium deposition (C) and adipogenic induction by Oil Red O staining for lipid droplets (D).

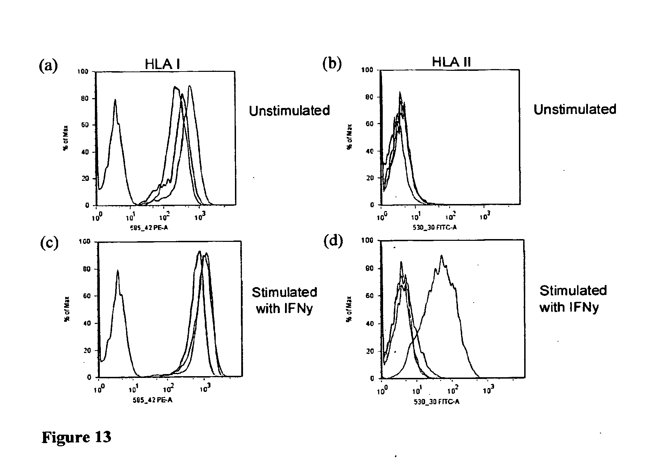

**Figure 13** **shows expression levels of cell surface (a,b) HLA I on OMLP PCs**
   Histograms demonstrating expression levels of cell surface (a,b) HLA I and (c,d) HLA II expression on OMLP-PCs and the changes in median flurorescence intensity in the presence/absence of IFNγ. Key: Red indicates Ig control, green day 1, blue day 2 and brown day 7.

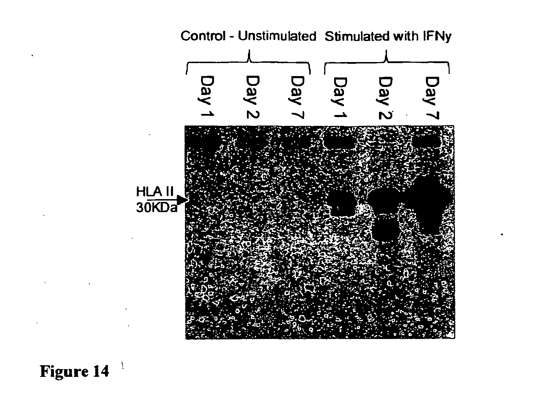

**Figure 14** **shows the absence of HLA II protein within unstimulated OMLP-PCs**
   Western blot demonstrating the absence of HLA II protein within unstimulated OMLP-PCs, and its induction after 24 hours with stimulation with IFNγ.

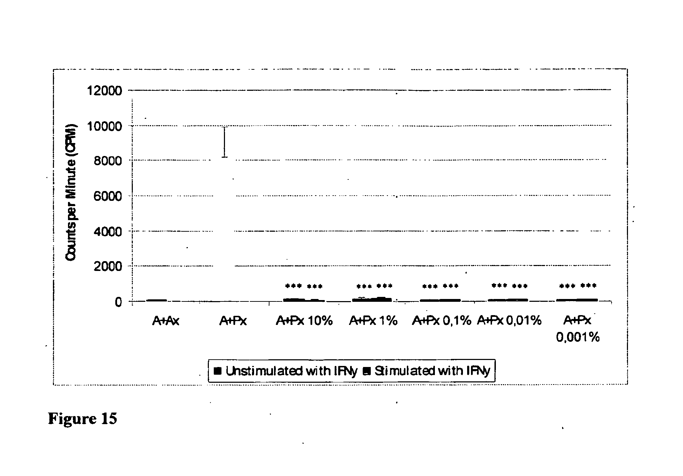

**Figure 15** **shows the dose-independent inhibitive effect of OMLP-PCs on lymphocyte proliferation.**
   One way MLC results demonstrating the potent inhibitive effect of OMLP-PCs on lymphocyte proliferation. These data demonstrate that this phenomenon is dose-independent and is irrespective of the induction of HLA II expression by pre-stimulation with IFNγ. Data are expressed as CPM +/- standard deviation. These data are from one OMLP-PC clone but are representation of all clones tested. *** P<0.001 as determined by one-way ANOVA with a Bonferroni post-hoc test.

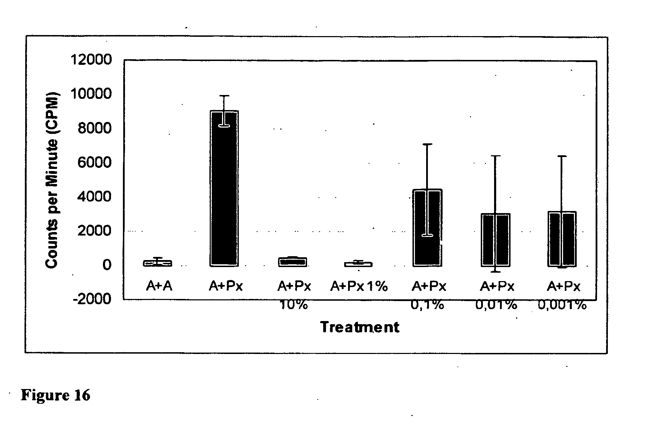

**Figure 16** **shows the variable inhibitive effect of MSCs on lymphocyte proliferation.**
   One way MLC results demonstrating the variable inhibitive effect of MSCs on lymphocyte proliferation. These data demonstrate that this phenomenon is dose-dependent. Data are expressed as CPM +/- standard deviation.

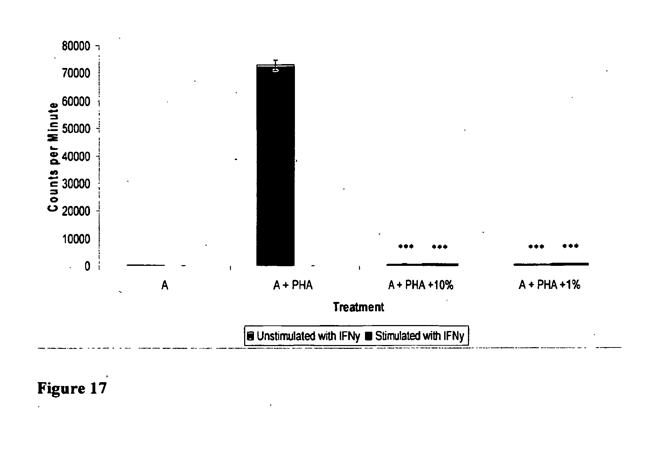

**Figure 17** **shows the dose-independent inhibitive effect of OMLP-PCs on T cell proliferation.**
   One way MLC results demonstrating the potent inhibitive effect of OMLP-PCs on T cell proliferation. These data demonstrate that this phenomenon is dose-independent and is irrespective of the induction of HLA II expression by pre-stimulation with IFNγ. Data are expressed as CPM +/- standard deviation. These data are from one OMLP-PC clone but are representation of all clones tested. *** P<0.001 as determined by one-way ANOVA with a Bonferroni post-hoc test.

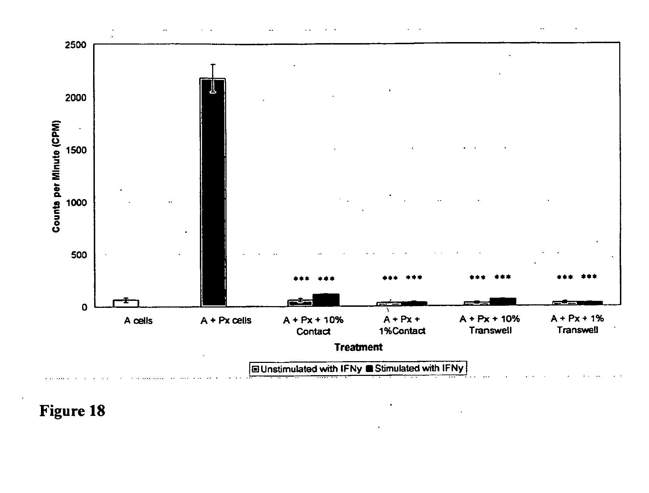

**Figure 18** **shows contact-independent inhibitive effect of OMLP-PCs on lymphocyte proliferation.**
   One way MLC results demonstrating the potent inhibitive effect of OMLP-PCs on lymphocyte proliferation in a contact-independent manner. These data demonstrate that this phenomenon is dose-independent and is irrespective of the induction of HLA II expression by pre-stimulation with IFNγ. Data are expressed as CPM +/- standard deviation. These data are from one OMLP-PC clone but are representation of all clones tested.
   *** P<0.001 as determined by one-way ANOVA with a Bonferroni post-hoc test.

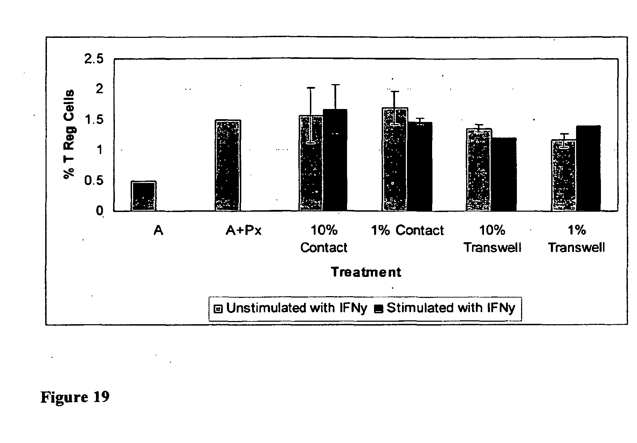

**Figure 19** **shows lack of effect of OMLP-PCs on number of T reg cells.**
   FACS results demonstrating no effect of OMLP-PCs on numbers of T reg cells. These data demonstrate that this phenomenon is dose-independent and is irrespective of the induction of HLA II expression by pre-stimulation with IFNγ. Data are expressed as average % T Reg cells +/- standard deviation. These data are from 3 patients.

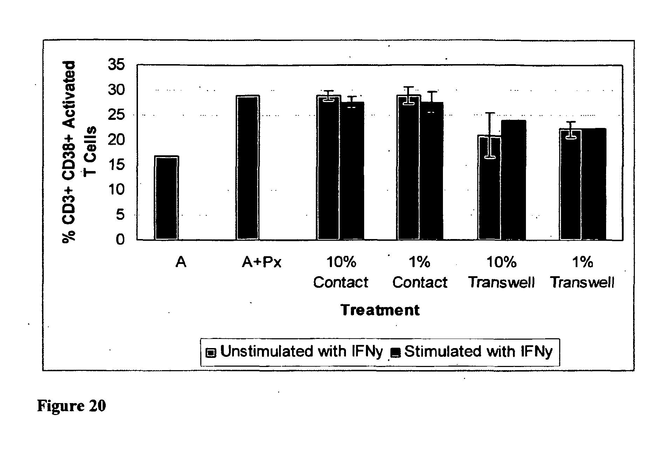

**Figure 20** **shows effect of OMLP-PCs on CD3+ CD38+ Activated T cells.**
   FACS results demonstrating the effect of OMLP-PCs on numbers of CD3+ CD38+ activated T cells when co-cultured in contact or in transwells with responder cells. These data demonstrate that this phenomenon is dose-independent and is irrespective of the induction of HLA II expression by pre-stimulation with IFNγ. Data are expressed as average % CD3+ CD38+ T cells +/- standard deviation. These data are from 3 patients.

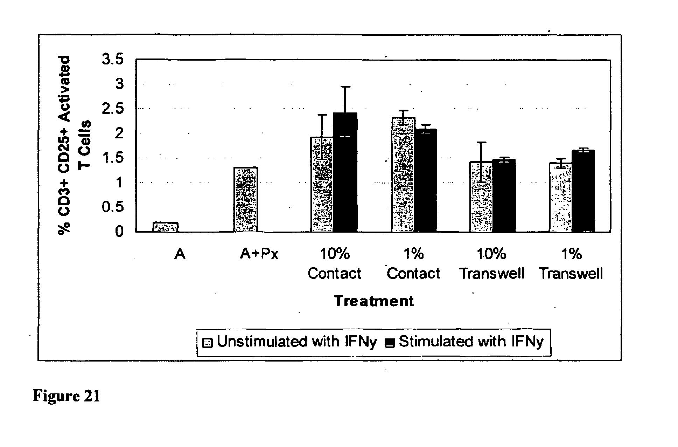

**Figure 21** **shows effect of OMLP-PCs on CD3+ CD25+ Activated T cells.**
   FACS results the effect of OMLP-PCs on numbers of CD3+ CD25+ activated T cells when co-cultured in contact or in transwells with responder cells. These data demonstrate that this phenomenon is dose-independent and is irrespective of the induction of HLA II expression by pre-stimulation with IFNγ. Data are expressed as average % CD3+ CD25+ T cells +/- standard deviation. These data are from 3 patients.

### Materials and Methods

### Isolation of Progenitor Cells and Maintenance in Culture

Normal, disease-free buccal mucosa biopsies were obtained from consented patients (n=3) undergoing routine dental procedures at the School of Dentistry, University Hospital of Wales, Cardiff University, UK. Local ethical committee approval had been previously obtained for this study and donors were informed in accordance with the Helsinki convention.

Single cell suspensions were obtained following enzymatic digestion of the biopsies as previously described (6). Tissues were surface sterilised in 70% Ethanol (30 secs), any excess fat was trimmed off the tissue and it was cut into 5mm² pieces. The tissue was then incubated overnight at 4°C with dispase (Sigma, UK; cat. no P-3417; 2mg/ml) to help separate epithelial and lamina propria tissue. The following day epithelial tissue was peeled off the lamina propria tissue and discarded. OMLP tissue was then mechanically minced and disaggregated overnight at 37°C with *Clostridium histolyticum* A collagenase (Roche Life Sciences, UK; cat. no 103 586; 1 mg/ml). Dispase and collagenase solutions were made up in serum containing media (SCM) [Dulbecco's modified Eagles medium [DMEM] (cat. no 21969-035) supplemented with 2mM L-glutamine, 100U/ml penicillin G, 100µg/ml streptomycin sulphate, 0.25µg/ml amphotericin B and 10% (v/v) heat inactivated foetal calf serum [FCS] (cat. no 10270-106). Media and supplements were purchased from Invitrogen, UK. Released cells were then pelleted, washed and pelleted again prior to resuspension in basal culture medium (Dulbecco's modified Eagles medium [DMEM] (cat. no 21969-035) supplemented with 2mM L-glutamine, 100U/ml penicillin G, 100µg/ml streptomycin sulphate, 0.25µg/ml amphotericin B).

PCs were separated by subsequent differential adhesion to fibronectin (10,15). OMLP single cell suspensions in basal culture media were seeded onto bovine plasma fibronectin (10µg/ml diluted in PBS+ [0.1 M phosphate buffered saline, pH 7.4, containing 1 mM magnesium chloride and 1 mM calcium chloride] overnight at 4°C; Sigma, UK; cat. no F-4759) coated 6 well plates and incubated for 20 minutes at 37°C. Non-adherent cells within this time frame were discarded and adhered cells were allowed to form single-cell colonies (>32 cells, eliminating any potential transit amplifying cells from our analysis) in SCM at 37°C in a 5% CO₂ humidified atmosphere. Cells within a given colony were manually counted before isolation by trypsinisation with 0.05 % (w/v) trypsin/0.53mM ethylenediaminetetraacetic acid (EDTA) (Invitrogen, UK; cat. no 25300-054) within a cloning ring and reseeded at a density of 2x10³ cells/cm² in SCM.

On reaching confluency, cells were passaged by trypsinisation using 0.05 % (w/v) trypsin/0.53mM ethylenediaminetetraacetic acid (EDTA) (Invitrogen, UK; cat. no 25300-054) and reseeded using the density described above. The population doublings (PD) of the cell populations *in vitro* were derived by direct counting of cell numbers at each passage as previously described (19). Clonal PC populations were cultured until senescence, as defined by a constant PD level/week of <0.5 and by assessment of cellular morphology.

### Heterogeneous expansion of clones

This method involved many of the techniques described above under the heading 'Isolation of Progenitor Cells and Maintenance in Culture'. However, following isolation of PCs by differential adhesion to fibronectin the adherent cells were allowed to grow to confluence before being passaged and expanded as a heterogeneous progenitor cell population on mono culture.

### RT-PCR for Embryonic and Developmental Markers

RNA was extracted from whole OMLP digests with Trizol® (Invitrogen, UK). cDNA was generated from 0.5µg of total RNA using random hexamer primers and Moloney murine leukaemia virus reverse transcriptase (Promega, UK) as per the manufacturer's instructions. Standard PCR reactions were carried out using GoTaq® (Promega, UK) as per the manufacturer's instructions with an initial denaturation step of 94°C for 5 minutes, followed by 40 cycles of 94°C (1 minute), Ta (30 seconds), 72°C (1 minute) with a final elongation step of 72°C for 10 minutes. cDNA was analysed for the expression of Nanog (17) [F 5' TGC TTA TTC AGG ACA GCC CT 3' and R 5' TCT GGT CTT CTG TTT CTT GAC T 3'] Ta 44°C , Oct4⁽²⁰⁾ [F 5' CGA CCA TCT GCC GCT TTG AG 3' and R 5' CCC CCT GTC CCC CAT TCC TA 3'] Ta 60°C, Sox2 [F 5'AAC CCC AAG ATG CAC AAC TC 3' and R 5'CGG GGC CGG TAT TTA TAA TC3'] Ta 55°C, KLF-4 [F 5'ACC CAC ACA GGT GAG AAA CC 3' and R 5'ATG TGT AAG GCG AGG TGG TC 3'], Ta 55°C, hTERT (21) [F 5'CGG AAG AGT GTC TGG AGC AA 3' and R 5' GGA TGA AGC GGA GTC TGG A 3'] Ta 55°C, Notch 1 [F 5' CTA CCT GTC AGA CGT GGC CT 3' and R 5' CGC AGA GGG TTG TAT TGG TTC G 3'] Ta 56°C, Notch 2 [F 5' AAG CAG AGT CCC AGT GCC TA 3' and R 5' CAG GGG GCA CTG ACA GTA AT 3'] Ta 56°C, Notch 3 [F 5' CAG TCG CCT GAG AAT GAT CAC 3' and R 5' GAA TGA CCA GCA GCA AGA CAG 3'] Ta 53°C, Delta 1 [F 5' ATC CTT GTC CTC ATG CTG CT 3' and R 5' GCC TTG AAG CCA TTC TTG TC 3'] Ta 53°C and Jagged 2 [F 5' CTA CAA TGG TGG CAT CTG TG 3' and R 5' GCG ATA CCC GTT GAT CTC AT 3'] Ta 53°C. Unpublished primers were designed using Primer3 primer design software (http://frodo.wi.mit.edu/cgi-bin/primer3/primer3 www.cgi). PCR products were separated on agarose gels and visualised with 0.005% (v/v) ethidium bromide. A positive control of HuES9 ESC line cDNA was run in all reactions.

### Flow Cytometry

Expanded cells for flow cytometry were passaged by treatment with Accutase™ (Sigma, UK) and counted before staining. Cell suspensions were washed in phosphate buffered saline (PBS) twice to remove residual serum before tri-labelling with the stem cell marker triplicate CD90- Allophycocyanin (APC; R & D Systems, UK), CD105- R-Phycoerythrin (RPE) and CD166-fluorescein isothiocyanate (FITC) (1:50 dilution of all antibodies; Ancell Inc., USA) for 45 minutes at 4°C. Cells were also separately stained using the same method for the haematopoietic and fibrocyte markers CD34-FITC and CD45-RPE (1:50; Ancell Inc., USA). Cells were washed twice in PBS before fixation in 2% paraformaldehyde for 5 minutes on ice. Fixed cell suspensions were diluted in 1ml of PBS before detection.
Cells were analysed on a FACSCanto™ flow cytometer (BD Biosciences, UK) equipped with a 488nm and 633nm laser excitation source with 30,000 events recorded per sample. Fluorophore conjugated immunoglobulins and single stains acted as respective controls. All data was compensated before analysis with FlowJo Version 7.2.2 software (Tree Star Inc., USA).

### Quantitative Telomeric Amplification Protocol (Q-TRAP)

The presence of active telomerase within expanded clones was analysed and quantified using an adapted Q-TRAP method to that previously described by Wege et al. 2003 (22). Briefly, cells were lysed in TRAP_{EZE}® 1X CHAPS lysis buffer (Chemicon International, UK) at a concentration of 10,000 cells/µl for 30 minutes on ice. Lysed cells were centrifuged for 20 minutes at 20,000xg at 4°C. The lysate supernatant was snap frozen using dry ice and stored at -80°C before use. A mastermix of 12.5µl Sybr Green PCR mix (Applied Biosystems, UK), 100ng of TS primer [5' AAT CCG TCG AGC AGA GTT 3'], 50ng ACX primer [5' GCG GCG CTT ACC CTT ACC CTA ACC 3'] (primer sequences previously published by Kim and Wu (1997) (23)) and diethylpyrocarbonate (DEPC) treated water totalling 20µl was made and an equivalent of 10,000 cells in 5µl was added per reaction. Heat inactivated (85°C for 30 minutes) lysates and lysis buffer were used as negative controls. A standard curve of 501 mel cell lysates (range 1-10,000 cells) was generated to calculate relative telomerase expression. All reactions were performed in triplicate using an AbiPrism 7000 sequence detection system (Applied Biosystems, UK) and data expressed as percentage relative telomerase expression to 10,000 cells of the 501 mel positive control cell line.

### Colony Forming Efficiency in the Presence of Soluble Jagged 1

Colony forming efficiency (CFE) was established using differential adhesion to fibronectin as described above. Colonies were allowed to form over 9 days in culture in SCM +/- the Notch ligand, recombinant rat Jagged 1/Fc chimera (R & D Systems, UK; 50ng/ml). Culture medium and Jagged 1 was replaced every 3 days.
CFE was expressed as a ratio of the total number of colonies formed (>32 cells) to the number of initially adherent cells.

### Immunocytochemistry (ICC)

*Developing Colonies:* Cells within developing colonies attached to fibronectin coated multi-well plates were fixed in ice cold methanol:acetone (1:1) for 20 minutes at 4°C. Cells were washed in PBS before permeabilisation with 0.1% Triton® X100 (Sigma, UK) at room temperature for 20 minutes (p75 nerve growth factor [NGF] receptor, Twist, Slug and Sox10 antibodies only). Fixed cells for telomerase staining were incubated with 2N hydrochloric acid for 20 minutes at room temperature before neutralising with 0.1 M sodium borate for 5 minutes as per the antibody manufacturer's instructions. Cells were subsequently blocked in appropriate serum (Dako UK Ltd., UK; 1:20 dilution) for 30 minutes at room temperature. Cells were incubated in primary antibody overnight at 4°C (p75 NGF receptor [ME20.4] 1:100, Twist [H-81] 1:50; Slug [G-18] 1:100; Santa Cruz Biotechnology Inc., Santa Cruz, CA; Snail [Ab17732] 1:50; Sox10 [Ab25978] 1:50; Telomerase [Ab5181] 1:2000; Abcam Plc, UK; Notch 1 [bTAN20] 1:5; Developmental Studies Hybridoma Bank, USA). Tetramethylrhodamine isothiocyanate (TRITC) anti-rabbit, FITC anti-mouse (Dako UK Ltd., UK), FITC anti-goat and FITC anti-rat (Sigma, UK) secondary antibodies were incubated for 1 hour at room temperature.

*Post neuronal and Schwann cell differentiation:* Cells were fixed, washed and blocked as previously described. Cells incubated with microtubule associated protein 2 (MAP2) antibody were permeabilised with 0.2% Triton® X100 before blocking. Cells were incubated in primary antibody overnight at 4°C (MAP2 [HM-2] 1:400; glial fibrillary associated protein (GFAP) [GF-5] 1:200; neuron specific β-III tubulin [TUJ-1] 1:1000; neurofilament M (NFM) [Ab9034] 1:500; S100 [4C4.9] 1:50; myelin basic protein (MBP) [EP1448Y] 1:100; Abcam Plc, UK). Secondary antibody incubation was as described above.
Where applicable nuclei were counterstained using a 4',6-diamidino-2-phenylindole (DAPI) containing mountant (Vector Laboratories, UK) and visualised using an Olympus Provis AX70 microscope (Olympus Optical Co., UK Ltd.) with a Nikon DXM1200 camera and Nikon ACT-1 software (Nikon UK Ltd.).

### Chondrogenic, Osteoblastic and Adipogenic Differentiation

Single cell suspensions of expanded clones were subject to differentiation induction protocols of multiple mesenchymal cell lineages.

*Chondrogenic Differentiation:* Single cell suspensions were pelleted at 1500 revolutions per minute (RPM) in microcentrifuge tubes at a concentration of 5x10⁵ per tube in 1 ml of SCM. Pellets were allowed to form overnight. SCM was replaced with chondrogenic induction media (basal media supplemented with 1 x insulin, transferrin and selenium (ITS) supplement (Invitrogen, UK), 50µg/ml L-ascorbate (Sigma, UK) and 5ng/ml transforming growth factor beta 1 (TGFβ1) (Peprotech EC Ltd., UK). Medium was changed every 2 days with pellets being centrifuged before aspiration of the conditioned media. Pellets were maintained in culture up to 21 days.

*Osteoblastic Differentiation:* Cells were seeded in 6 well cultures dishes at a concentration of 3x10³ cells/cm². Cells were cultured in SCM until confluency was reached whereby the media was replaced with osteogenic induction media (Alpha modified Eagles medium (α-MEM) (Invitrogen, UK) supplemented with 10% (v/v) FCS, 2mM L-glutamine, 100U/ml penicillin G, 100µg/ml streptomycin sulphate, 0.25µg/ml amphotericin B, 50µg/ml L-ascorbate, 10µM dexamethasone and 5mM beta-glycerophosphate (Sigma, UK)). Cultures were fed with osteogenic induction medium every 3 days and cultured for a total of 21 days.

*Adipogenic Differentiation:* Cells were seeded in 6 well cultures dishes at a concentration of 3x10³ cells/cm². Cells were cultured in SCM until confluency was reached whereby the media was replaced with adipogenic induction media (SCM supplemented with 10µg/ml insulin, 1µM dexamethasone, 100µM indomethacin, 100µM 3-isobutyl-1-methyl-xanthine [IBMX] (Sigma, UK)) for 3 days. Cells were then incubated with adipogenic maintenance medium (SCM supplemented with 10µg/ml) for a further 1 day. Cells were cultured in repeating induction and maintenance cycles for a total of 28 days.

### Neuronal and Schwann Cell Differentiation

Isolated colonies were cultured and expanded in a defined ESC media (24). Colonies were trypsinised as previously described and seeded onto human laminin coated multi-well plates (2µg/cm²; Sigma, UK). Cells were maintained in X-VI\/O™10 medium (Lonza Group Ltd., UK) supplemented with 0.1 mM β-mercaptoethanol (Sigma, UK), 1% (v/v) non-essential amino acids (Invitrogen, UK), 100U/ml penicillin G, 100µg/ml streptomycin sulphate, 0.25µg/ml amphotericin B and 80ng/ml recombinant human basic fibroblast growth factor (rhbFGF) (Peprotech EC Ltd., UK).

Cells were passaged and neurospheres disaggregated by incubation in 1mg/ml *Clostridium histolyticum* collagenase IV (Invitrogen, UK) for 5-10 minutes at 37°C and subsequent mechanical disaggregation.

Cells were subjected to neuronal differentiation using previously described protocols (16,17). Briefly, single cell suspensions were counted and seeded onto 0.1% Matrigel™ (BD Biosciences, UK) coated chamberslides at 5.6x10³ cells/cm² in DMEM-F12 (3:1) media supplemented with 40ng/ml hrbFGF, 10% (v/v) FCS, 2mM L-Glutamine, 100U/ml penicillin G, 100µg/ml streptomycin sulphate and 0.25µg/ml amphotericin B. Cells were cultured for 7 days with media changes every 3 days. After 7 days in culture, the hrbFGF was removed from the culture media and replaced with 10ng/ml nerve growth factor (NGF), 10ng/ml brain derived growth factor (BDNF) and 10ng/ml neurotrophin 3 (NT3). Cells were cultured for an additional 7 days with media changes every 3 days.

Schwann cell differentiation was as previously described by Toma et al., (2005) (18). Collagenase treated cells were seeded onto poly-D-lysine laminin coated chamberslides (2µg/ml) at an equivalent density to that described for neuronal differentiation. Cells were cultured in DMEM-F12 (3:1) with 10% (v/v) FCS, 2mM L-Glutamine, 100U/ml penicillin G, 100µg/ml streptomycin sulphate and 0.25µg/ml amphotericin B for 7 days. Media was then replaced with neurobasal media (Invitrogen, UK) containing 1% (v/v) FCS, 1% (v/v) N2 supplement (Invitrogen, UK), 4µM forskolin (Sigma, UK), 2mM L-Glutamine, 100U/ml penicillin G, 100µg/ml streptomycin sulphate, 0.25µg/ml amphotericin B and 10ng/ml heregulin β1 (Peprotech EC Ltd., UK). Cells were maintained in this media for a further 7 days. Media was changed every 3 days, with 50% of the conditioned media being reapplied to the cells.

### Immunohistochemistry

10µm cryosections were taken of chondrogenically differentiated cell pellets after 21 days in culture. Sections were washed in PBS for 10 minutes before blocking in goat serum (1:20; Dako UK Ltd., UK) for 30 minutes at room temperature. Sections were incubated in aggrecan antibody [1:50; 6B4] (a generous gift from Professor Bruce Caterson, Cardiff University) overnight at 4°C in a humidified chamber. Slides were subsequently washed in PBS for 10 minutes and incubated with an anti-mouse secondary antibody (1:50; Dako UK Ltd., UK) for 1 hour at room temperature. Sections were washed in PBS before mounting under coverslips with fluorescence mounting medium (Dako UK Ltd., UK). Slides were visualised as previously described for ICC.

### Histology

*Van Gieson's staining of chondrogenic pellets:* Cryosections cut from chondrogenically differentiated pellets were prepared as previously described. Sections were washed in water before staining with Celestin blue for 5 minutes. Sections were rinsed with water and stained for a further 5 minutes with Mayer's haematoxylin. Sections were washed in water and differentiated in 1% (v/v) hydrochloric acid in 70% (v/v) ethanol. Sections were washed in running water for a further 5 minutes before staining with Van Gieson's stain for 3 minutes. Slides were washed again before mounting under a coverslip with DPX mountant (Sigma, UK).

*Von Kossa Staining for Osteoblastic Differentiation:* Differentiated cells were fixed in 70% (v/v) ethanol overnight at 4°C. The cells were washed in PBS before incubation with 5% (w/v) silver nitrate (Sigma, UK) for 5 minutes in the dark. After washing, the cells were incubated in 5% (w/v) sodium carbonate containing 10% (v/v) formaldehyde solution for 2 minutes. The cells were copiously washed in water for 15-20 minutes before incubation in Farmers reducer (0.1% (w/v) sodium thiosulphate and 0.1% (w/v) potassium ferricyanide) for 30 seconds. Cells were washed for a further 15-20 minutes in water before visualisation.

*Oil Red O Staining for Adipogenic Differentiation:* Differentiated cells fixed in 70% ethanol were rinsed in 60% (v/v) isopropyl alcohol (Sigma, UK) for 5 minutes at room temperature with agitation. Cells were stained with Oil red O (0.25% (w/v) Oil red O (Sigma, UK) in isopropyl alcohol) for 10 minutes at room temperature with agitation. Cells were briefly rinsed in 60% (v/v) isopropyl alcohol before washing with water and visualising.

### RT-PCR for Adipogenic Markers

RNA was extracted from adipogenic differentiated OMLP-PCs with Trizole® (Invitrogen, UK). cDNA was generated from 0.5µg of total RNA using random hexamer primers and Moloney murine leukaemia virus reverse transcriptase (Promega, UK) as per the manufacturer's instructions. Standard PCR reactions were carried out using GoTaq® (Promega, UK) as per the manufacturer's instructions with an initial denaturation step of 94°C for 5 minutes, followed by 40 cycles of 94°C (1 minute), Ta (30 seconds), 72°C (1 minute) with a final elongation step of 72°C for 10 minutes. cDNA was analysed for the expression of lipoprotein lipase [F 5' CCT GCT CGT GCT GAC TCT GG 3' and R 5' CAT CCT GTC CCA CCA GTT TGG 3'], CAATT/Enhancer binding protein alpha [CEBPα] [F 5' CCG GCC TCT TCC CTT ACC AG 3' and R 5' CCA CCG ACT TCT TGG CCT TG 3'] and peroxisome proliferator-activated receptor gamma [PPARγ] [F 5' CCA CAG GCC GAG AAG GAG AA 3' and R 5' CCA GCA GCC CTG AAA GAT GC 3']. Primers were designed using Primer3 primer design software (http://frodo.wi.mit.edu/cgi-bin/primer3/primer3 www.cgi). PCR products were separated on agarose gels and visualised with 0.005% (v/v) ethidium bromide.

### Immunomodulatory Activity

### Cell Culture of OMLP-PCs

OMLP-PC clones previously established were grown in monolayer in serum containing media (SCM) as previously described at 37°C in 5% CO₂. For clones requiring stimulation with IFNy, cells were grown to 60% confluency before stimulation with SCM supplemented with 100U/ml of IFNγ. Cells were maintained in said media for 1,2 or 7 days in culture. Media was changed and cells re-stimulated with fresh IFNy at day *3.FACs Analysis of OMLP-PCs for Extracellular HLA I and II Expression*
OMLP-PCs were grown in the presence/absence of 100U/ml IFNγ and assayed by FACS analysis for HLA I and II expression at days 1, 2 and 7 days post-stimulation.
Conditioned media was removed from the cells and stored at -80 °C for future analysis. The cells were washed with PBS before being detached from the plastic using Accutase™ (Sigma, UK) and washed again in PBS. Cells were centrifuged at 1700 RPM for 5 minutes, the supernatant removed and the cells resuspended in 0.1% bovine serum albumin (BSA) in PBS. This step was repeated once. Cells were incubated with HLA-I or HLA-II antibodies (1:50 dilution; Dako, UK) for 15 minutes at room temperature in the dark. Subsequent to incubation with the antibodies, cells were spun down once more and washed in 0.1% BSA in PBS. The cells were pelleted again and then resuspended in 200ul of 0.1% BSA in PBS for analysis on the FACSCanto™ flow cytometer (BD Biosciences, UK) as previously described.

### Western Blotting for Analysis of Intracellular and Extracellular HLA I and II Expression

OMLP-PCs were grown in the presence/absence of 100U/ml IFNγ and analysed by Western blotting for HLA I and II expression at days 1, 2 and 7 post-stimulation. The cells were washed in PBS before being lysed in 1% Triton@ X-100/0.25M sucrose containing Complete™ protease inhibitors (Roche Applied Science, UK). Protein extracts were sonicated to release intracellular proteins before quantification using the BCA protein assay (Pierce, UK).
Protein extracts (5ug) were run on 12% acrylamide resolving gels to separate proteins and then transferred to nitrocellulose membrane overnight at 30V at 4°C. Membranes were blocked with 5% non-fat dry milk (Biorad, UK) in Tris-buffered saline (TBS) containing 0.1% Tween® 20 (TBS-T), washed 3 times in PBS containing 0.1 % Tween® 20 (PBS-T) and incubated with a HLA II antibody (1:250 dilution in 2% non-fat dry milk; Dako, UK) for 1 hour at room temperature. The membrane was washed three times in PBS-T before incubation with an anti-mouse horse-radish peroxidise (HRP) conjugated secondary antibody (1:3000 diluted in 2% non-fat dry milk; Biorad, UK) for 1 hour at room temperature. The membrane was subsequently washed twice in PBS-T and once in PBS before detection of the proteins by ECL™ detection (GE Healthcare, UK).

### Determining the Effect of OMLP-PCs on Lymphocyte Proliferation by Mixed Lymphocyte Culture (MLC)

OMLP-PCs were grown in the presence/absence of 100U/ml IFNγ and assayed by MLC at 7 days post-stimulation. The cells were washed with PBS before being detached from the plastic using Accutase™ (Sigma, UK) and washed again in RPMI media containing 10% human AB serum, 2mM L-glutamine, 100U/ml penicillin G, 100µg/ml streptomycin sulphate, 0.25µg/ml amphotericin B. The cells were spun at 1700 RPM for 5 minutes and resuspended in the above RPMI media for counting. Cells were gamma irradiated with 20Gy for setting up of the MLC.

Peripheral blood lymphocytes (PBLs) were prepared as previously described (25). Briefly, PBLs were purified from heparinised blood by Ficoll-Hypaque gradient for 20 minutes at 600 x g. Responder PBLs (A) were incubated with an equal number of irradiated stimulator PBLs from a pool of donors (Px) or an autologous control (Ax) in the above RPMI media. Irradiated OMLP-PCs were added to the cultures at a ratio of 100%-0.001% to responders and incubated for 5 days at 37°C at 5% CO₂. Cultures were frozen on day 6 after 24 hours of tritiated thymidine incorporation (1 uCi). Cultures were defrosted and proliferation recorded on day 7. Cells were harvested automatically on a glass fibre filter with a TomTec harvesting machine (Harvester 96, Tomtec, Orange, CT, USA).

### Determining the Effect of Lymphocyte Proliferation in the Presence of T cell Mitogens by Mixed Lymphocyte Culture

Standard mixed lymphocyte culture (MLC) MLCs were set up as described above, with OMLP-PCs added at a 1:1 to 0.01:1 ratio to responder cells. The T cell mitogen, phytohaemagglutinin (PHA) was added to cultures at 10ug/ml. Proliferation was recorded on day 4 as described above.

### Determining Whether the OMLP-PC Induced Immunosuppression is Contact Dependent

MLCs were set up as previously described in 12 well plate format. OMLP-PCs were added to cultures at either a 0.1:1 to 0.01:1 ratio to responder cells. Contact cultures involved OMLP-PCs being added directly to the MLC culture. Transwell cultures involved the OMLP-PCs being seeded onto inserts with a 0.4um pore-sized membrane. This separates the cells from direct contact with the responder cells, but ensures that they share the same media. Cultures were maintained for 5 days, before PBLs were removed and replated into 96 well plates and stimulated with tritiated thymidine for 24 hours at 37°C. Proliferation was recorded as previously described.
A proportion of the PBLs were removed for FACS analysis as described below.

### FACS Analysis of Stimulated PBLs for T Regulatory Cell and T Cell Activation Markers

PBLs were removed from the transwell and contact studies above were spun down at 1700 RPM for 5 minutes. The supernatant was removed and stored at -80 °C for future analysis. Cell pellets were resuspended and washed in 0.1 % BSA in PBS. Cells were prepared for FACS as previously described for HLA I and II detection. Cells were quad-labelled for CD38, CD3, CD25 and CD4 or CD25, CD3, CD127 and CD4. Autologous controls and A+ Px cell incubations were used as controls.

### Results

### Expression of Embryonic and Developmental Markers within the OMLP Suggests the Presence of a Progenitor Cell Population

RT-PCR confirmed the positive expression of the embryonic and pluripotency markers Nanog and Oct4 within whole OMLP tissue digests. Expression of human reverse transcriptase (hTERT) was also detected in tissue isolates suggesting the presence of a telomerase positive subpopulation of cells resident within the OMLP. The iPS markers Sox2 and KLF-4 (as well as Nanog and Oct4) were also present within the whole tissue. Developmental markers associated with the Notch signalling pathways, the Notch receptors 1, 2 and 3 and the Notch ligands Delta 1 and Jagged 2 were also positively expressed within whole OMLP digests suggesting the presence of immature cell types and ongoing Notch signalling (Fig 7).

### A PC Population can be Reliably Isolated from the OMLP and Undergoes Rapid Proliferation In Vitro

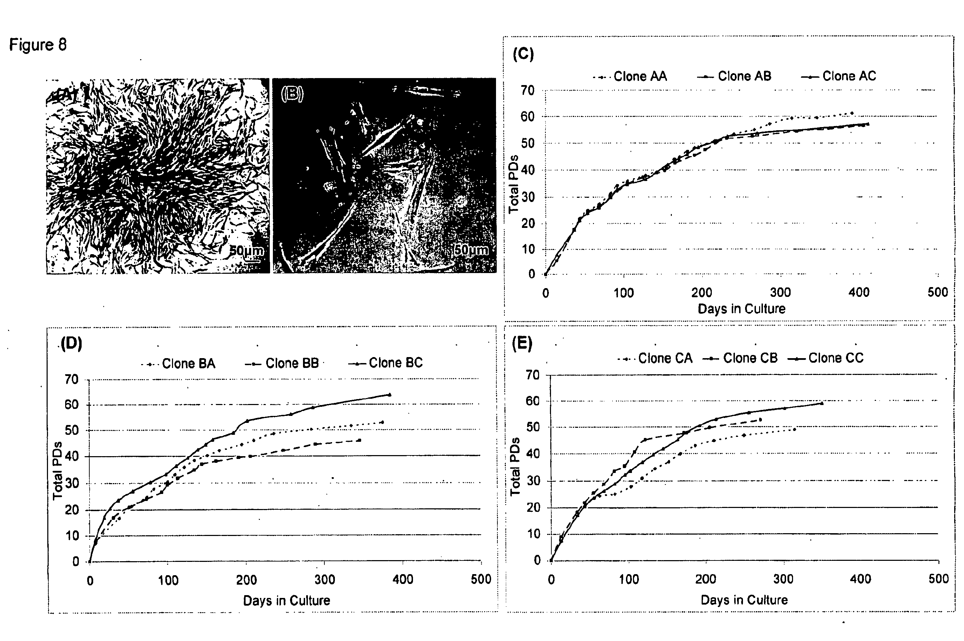

PCs were readily isolated from the OMLP, in our example, by differential adhesion to fibronectin. As previously reported, one attribute of stem and progenitor cell populations is their ability to self-renew by the formation of colonies originating from a single cell. This OMLP fibronectin adherent cell population successfully generated clones from single adherent cells and demonstrated a fibroblast-like morphology, bi-polar in nature in developing colonies and expanded clones (Figs 8A&B). This cell population was characterised by a high initial rate of proliferation (on average >4 PDs/week), before establishing a constant level of 1 PD/week and reaching cellular senescence at approximately 50-60 PDs (Figs 8C-E). These growth kinetics are representation of all clones isolated and expanded from all patients.

### OMLP PCs Express Cell Surface Stem Cell Markers

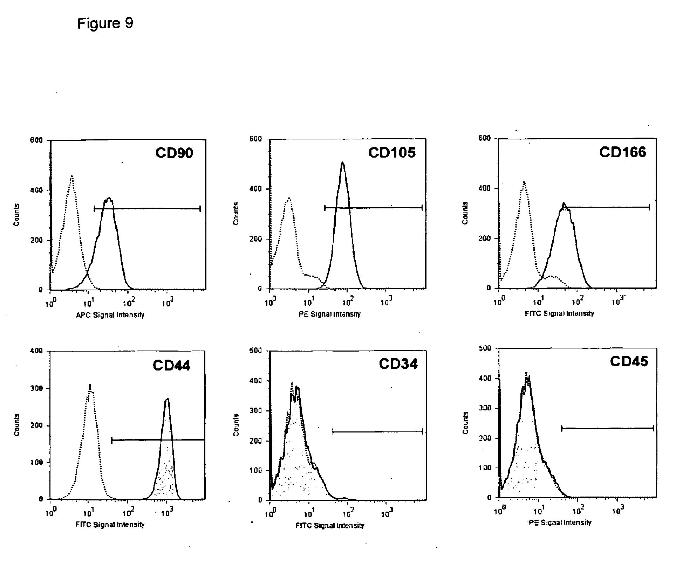

Clones were expanded in monolayer culture before determining the expression of the stem cell marker series STRO-1, CD44, CD146, CD90, CD105, CD166 and the haematopoietic and fibrocyte markers CD34 and CD45,using the flow cytometry methods described herein. All clones from all patients tested demonstrated positive expression of STRO-1, CD44, CD146, CD90, CD105 and CD166 but were negative for CD34 and CD45 (Fig 9, Data not shown for STRO-1 and CD146).

### OMLP PCs Express Active Telomerase

Telomerase activity is generally considered to be diminished within somatic cells with the exception of stem cells and lymphocytes. In contrast human mesenchymal stem cells (MSCs) have been shown to lack activity for telomerase despite their stem cell properties (3). Telomerase expression within developing OMLP colonies was assessed by ICC and within expanded clones from all patients by Q-TRAP.

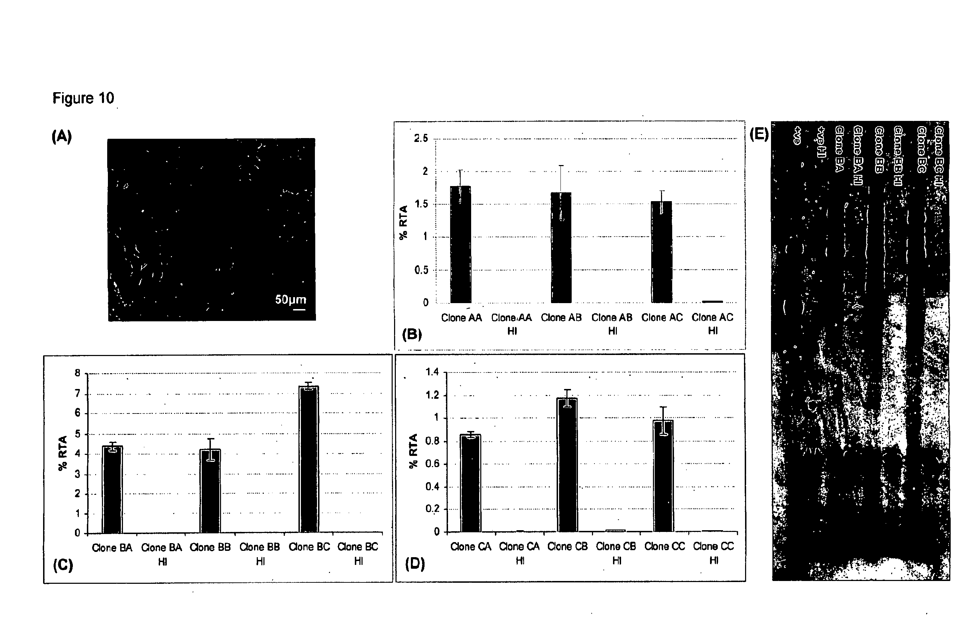

Positive expression of telomerase was demonstrated within all the developing colonies tested (Fig 10A) and was maintained within the expanded clones (Fig 10B-D). Separation of the Q-TRAP products on acrylamide gels confirmed the typical 'step-ladder' effect observed with telomeric repeat amplification (Fig 10E). Relative levels of telomerase within expanded clones were patient specific, but did not vary greatly between clones of the same patient, correlating with the small differences between total population doublings (PDs) of patient matched clones reaching cellular senescence (Fig 8B).

### OMLP PCs Originate from the Neural Crest

Attentuation of Notch signalling, through soluble Jagged 1 or γ-secretase inhibitor exposure, has been demonstrated to have differential effects on the proliferation of MSCs and neural crest stem cells (NCSCs). The γ-secretase inhibitor N-[N-3,5-Difluorophenacetyl-L-alanyl]-S-phenylglycine t-butyl ester (DAPT) has been previously reported to slow MSC growth, eventually resulting in inhibited cell division. In contrast, exposure of NCSCs to soluble Jagged 1 inhibits neuronal differentiation of the cells and therefore allowing increased proliferation and expansion.

Colony formimg efficiency (CFE) of OMLP PCs in the presence of soluble Jagged 1 was found to be significantly higher (P<0.05) than CFE within control media (Figs 11A-B), supporting results observed within NCSCs and thus suggesting a neural crest origin for this PC population. To further confirm a neural crest origin for OMLP PCs, the production of defined neural crest markers and the developmental marker Notch 1 were investigated by ICC within developing colonies. The transcription factors Sox10, Snail, Slug and Twist were positively localised primarily to the cell nuclei, but were also found within the cytoplasmic compartment (Figs 11C-G). Notch 1 was identified within the cytoplasm of the PCs. These data further support a neural crest origin for this PC population.

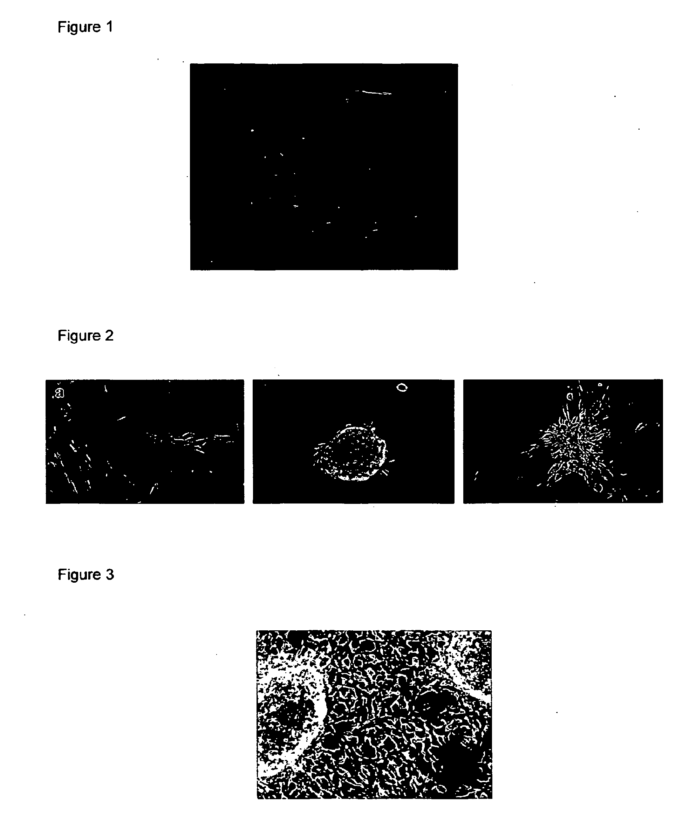

Expression of p75 nerve growth factor (NGF), an additional neural crest marker was also found within cells isolated by differential adhesion to fibronectin (Fig 1). Positive expression of this marker was found within cells adhered to the top of developing colonies. These cells demonstrated a considerably different morphology to those cells within developing colonies. These data suggest that in addition to the p75- cell population herein characterised, there also exists a p75+ cell population which remains uncharacterised.

### OMLP PCs are Multipotent and can Generate Cells of Multiple Lineages

OMLP PCs from multiple clones were differentiated down cell lineages of both mesenchymal and neuronal origin.

Evidence of chondrocyte differentiation was demonstrated by collagen and aggrecan production with pellet cultures (Fig 12A&B). Mineralisation was observed within osteoblastic differentiated PC cultures (Fig 12C).Lipid droplets were observed in adipogenic stimulated cultures utilising Oil Red O staining and RT-PCR tests for the markers lipoprotein lipase, CEBP-α and PPARγ were positive (Fig 12D; data not shown for RT-PCR). These data confirm that OMLP PCs are multipotent and are capable of generating mesenchymal cell types osteoblasts, chondrocytes and adipocytes as previously demonstrated for stem cells isolated from multiple sites such as the bone marrow and dental pulp.

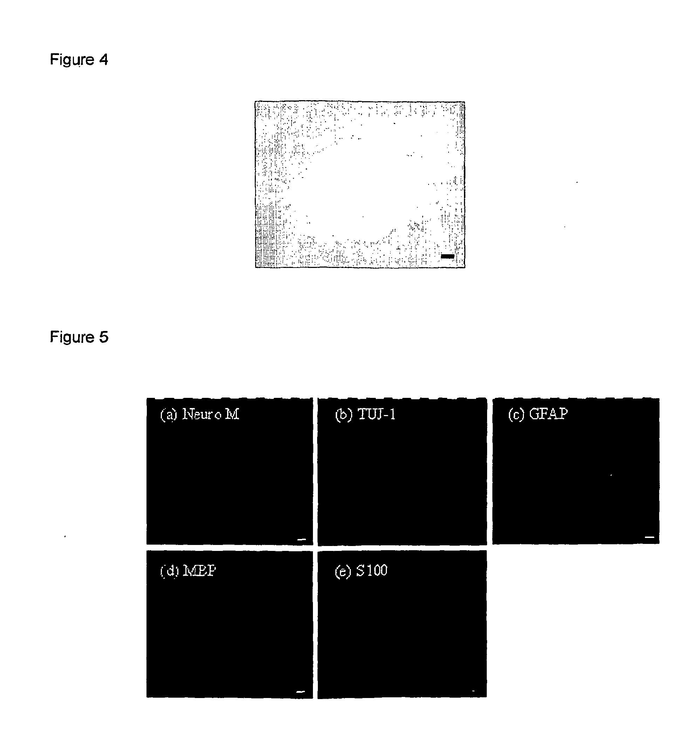

Neuronal and Schwann cell differentiation was not possible with PCs which had been serially expanded in monolayer within SCM *(data not shown).* In order to positively induce neuronal and Schwann cell differentiation, clones were expanded in monolayer on laminin coated plates in the presence of defined ESC media. The morphology of the PCs changed with incubation in these defined conditions with the formation of distinct neurosphere-like bodies (Figs 2A and 4) similar to that seen with cells from the SY5Y neuroblastoma cell line (Fig 3). Utilising defined induction media successful differentiation of the expanded clones was possible down the neuronal cell lineage as detected by positive expression of the neuronal markers Nestin, TUJ-1 (β-III tubulin), NFM, GFAP and MAP2 by ICC (Figs 6B-F) and also down the Schwann cell lineage as detected by the positive expression of the Schwann cell specific markers MBP and S100 (Figs 6G&H). Only TUJ-1 (β-III tubulin), GFAP and S100 were expressed by clones prior to differentiation, although this was to a much lesser extent than the levels seen upon differentiation (Fig 5B, C and E).

### OMLP PCs are Immunomodulatory

### OMLP-PCs Express HLA I but not HLA II on their Cell Surface

FACS analysis confirmed that unstimulated OMLP-PC clones expressed HLA I but no HLA II on their cell surface (Figs 13a & b). Stimulation with 100U/ml IFNγ upregulated HLA I expression after 24 hours, but did not induce cell surface expression of HLA II until day 7 (Figs 13c & d). These results were consistent over the 3 clones from each of the 3 patients analysed.

### OMLP-PCs Express Intracellular HLA II After 24 hours of Stimulation with IFNγ

Western blot analysis was performed of total protein extracts from OMLP-PC clones incubated for 1, 2 or 7 days in the presence/absence of 100U/ml IFNγ. Blots confirmed that no expression of HLA II was detected extra- or intracellularly in unstimulated OMLP-PCs (Fig 14). Stimulation with IFNγ, induced intracellular expression of HLA II after 24 hours (Fig 14), but from the FACS results described above we can assume that this protein is not transported to the cell membrane until day 7. These data are representation of all the clones and patients tested.

### OMLP-PCs are Potently Immunosuppressive and can act Directly on T Cell Proliferation

Standard mixed lymphocyte culture (MLC) cultures demonstrated the massive proliferative effect on responder cells seen with incubation with pool lymphocytes from other multiple donors. Addition of OMLP-PCs to the culture inhibited this proliferative effect back to base levels seen in autologous control cultures.

Interestingly, this effect was seen to be dose-independent, with 98-99% inhibition of lymphocyte proliferation being demonstrated, regardless of whether OMLP-PCs were added at a ratio of 10% or 0.001 % to responder cells (Fig 15). Similar levels of inhibition were also seen with OMLP-PCs pre-stimulated for 7 days with IFNγ to those cells unstimulated, demonstrating that this effect is independent of HLA II expression (Fig 15).

When directly compared with cultures using mesenchymal stem cells (MSCs) as the immunosuppressive stimulus, a dose-dependent effect on lymphocyte proliferation was demonstrated (Fig 16). Where 95% of lymphocyte proliferation was achieved using a 10% ratio of MSCs, only 65% inhibition was seen when cell numbers were lowered to 0.001%. This equates to OMLP-PCs being on average 33 fold more efficient at inhibiting lymphocyte proliferation at a 0.001% ratio to responder cells. In addition huge variability between repeats was seen with the MSC cultures, in comparison to accurate repetitions seen with the OMLP-PC clones.

MLC cultures performed in the presence of the T cell mitogen, PHA, demonstrated that OMLP-PCs are able to reduce T cell proliferation directly back to basal levels, irrespective of pre-stimulation with IFNγ (Fig 17). These results confirm that OMLP-PCs can act directly on T cells, although whether this is by the same mechanism as that seen in the inhibition of lymphocytes in the one-way MLCs described above remains unclear.

### OMLP-PCs are Immunosuppressive Using a Contact Independent Mechanism

One-way MLCs were performed using OMLP-PCs in direct contact or in transwell culture with responder cells. The data obtained demonstrates the potent immunosuppressive action of the OMLP-PCs, with no significant difference between lymphocyte proliferation with cells in direct contact with the PCs compared with those not in direct contact (Fig 18). Complete inhibition of lymphocyte proliferation was seen back to base levels regardless of treatment. This effect was also independent of the concentration of OMLP-PCs used within the assay and irrespective of pre-stimulation of the cells with IFNγ before use within the MLC assay.

### OMLP-PC mechanism of immunosuppression may be dependent on culture method

FACS analysis of peripheral blood lymphocytes (PBLs) recovered after MLC in contact and transwell co-cultures with OMLP-PCs determined the levels of subpopulations of T cells. CD3+, CD4+ and CD127 low expressing T cells, known as T reg cells, were demonstrated to triple in number on incubation with pool donor lymphocytes. These numbers did not appear to be changed with the addition of OMLP-PCs to the cultures regardless of pre-stimulation with IFNγ or contact (Fig 19).

Changes in levels of activated T cells was subcategorised into mature CD3+ T cells expressing C38+ and CD3+ , CD25+ (IL-2 receptor) and analysed. Interestingly, although no difference in the level of immunosuppression was seen with respect to cell-cell contact, a decrease in the level of activated CD3+, CD38+ T cells was seen within the transwell culture system (Fig 20) and an induction of CD3+ CD25+ T cells were seen within the contact system (Fig 21). These effects were independent of pre-stimulation with IFNγ. Statistics have not been performed as figures generated are average percentages from 3 patients.

### Discussion

ASC populations have become an attractive source of multipotent undifferentiated cells for use in numerous tissue engineering applications. They hold distinct advantages over ESCs and induced pluripotent stem cells (iPS) due to their ability to rapidly expand *in vitro* but still to a finite level limiting fears of potential tumourogenic implications on transplantation. Furthermore, these cells are multipotent, readily differentiate upon stimulation and there are no current ethical or legal issues regarding the use of adult sources of stem cells.

In this study we have demonstrated for the first time, the existence of a novel PC population resident within the OMLP. These cells can be clonally expanded from a single cell *in vitro,* generating a homogenous population of cells for accurate characterisation. This enrichment process offers distinct advantages in generating and expanding sufficient cell numbers of ASC populations for therapeutic applications. We have specifically used clonal populations of cells (rather than heterogenous isolations from unsorted whole tissue) for this characterisation study as this allows accurate definition of a pure cell population. It also gives a far more accurate indication as to how these cells would respond and differentiate upon transplantation compared to unsorted or whole tissue isolates. The advantages of stem cell/clonal sorting has been previously demonstrated by Kotobuki et al., (2004) who demonstrate the enhancement of osteoblastic potential of sorted osteoblast-like cells for bone tissue engineering applications (26).

Clonally expanded OMLP PCs are ideally characterised by the positive expression of STRO-1, CD44, CD146, CD90, CD105 and CD166, but are CD34 and CD45 negative, ruling out a haematopoietic or fibrocyte origin. These data also confirm a similar expression profile for OMLP PCs to bone marrow mesenchymal stem cells (27). Expansion in monolayer culture has confirmed that these cells will undergo a period of rapid proliferation, but have a finite lifespan entering replicative senescence after extensive population doublings (>50PDs). In contrast to MSCs (3), OMLP PCs demonstrate the expression of active telomerase within both developing colonies and also after extensive passaging, providing further support for the stem cell phenotype of the isolated OMLP cells.

Increased CFE in the presence of the Notch ligand, soluble Jagged 1 and the expression of neural crest markers Slug, Snail, Twist and Sox10 within developing colonies adherent to fibronectin confirm the neural crest origin of these cells. The craniofacial location of these cells *in vivo* would suggest that this cell population arises from the cephalic neural crest region evidence supported by the plasticity of this cell population to generate cell lineages of both mesenchymal and ectodermal origin including osteoblasts, chondrocytes, adipocytes, neurons and Schwann cells. Interestingly the multipotency and profile of lineages OMLP PCs can generate is comparable to that of defined NCSCs within the craniofacial region.

Our data succinctly demonstrate the potent immunomodulatory capacities of OMLP-PCs.

Within the work discussed above we have demonstrated that OMLP-PCs, like both adult and foetal MSCs, express HLA I but no HLA II on their cell surface (28,29). Differences between expression levels in HLA I and II within foetal and adult MSCs arise on stimulation of the cells with IFNγ. Here adult MSCs are shown to upregulate their cell surface expression of HLA II within 24-48 hours of incubation with IFNγ (29), however 7 days of stimulation are required to induce cell surface expression of HLA II within foetal MSCs. Within OMLP-PCs we demonstrated a foetal MSC like response to IFNγ stimulation, with cell surface expression not seen until day 7. In contrast intracellular levels of HLA II were detectable by Western blotting within 24 hours of stimulation within OMLP-PCs. This is a similar response time to that reported within both adult and foetal MSCs (28,29).

Within MLC cultures OMLP-PCs were demonstrated to have a potent immunosuppressive effect on responder lymphocytes, inhibiting lymphocyte proliferation back to basal levels. Cells were demonstrated to be 98-99% effective in this response, and this effect was independent of the ratio of OMLP-PCs used to responder cells and also pre-stimulation for 7 days with IFNγ to induce the expression of HLA II. These results demonstrate the efficiency by which these cells are able to inhibit lymphocyte proliferation and that their method of action is not through the classical presentation pathway. In contrast, comparative experiments conducted with adult MSCs demonstrated the dose-dependent immunosuppressive mode of action by these cells and their lower efficiency - 33 fold lower at the lowest cell:cell ratio used.

These results also demonstrate the clear advantage of working within a clonal cell population for translation to clinical applications. Replicates between clones and patients were highly homologous with the OMLP-PCs, however huge variability was seen within the MSC cultures, which contain a heterogenous population of cells (this is seen by comparing the data in figures 15 and 16).

Using the T cell mitogen PHA we have demonstrated the direct effect OMLP-PCs can have on T cells, inhibiting their proliferation, and using transwell culture techniques demonstrated that immunosuppressive effects seen in routine MLC conditions are contact-independent and independent of induction of HLA II cell surface expression. FACS analysis of responder cells utilised within the contact and transwell experiments demonstrated that although no difference in levels of immunosuppression was seen in contact or transwell cultures, differences in levels of activated T cells were demonstrated between the 2 treatment groups. Induction of CD3+ CD38+ T cells within contact cultures and suppression of CD3+ CD25+ T cells within transwell studies suggests differential modes of action between OMLP-PCs in direct contact with responder cells to those within the transwell system. The later acting directly through one or more soluble factors being released into the culture media and affecting the T cells.

OMLP-PCs are able to act in a dose-independent manner and independently of HLA II expression on their cell surface or intracellulary. These effects occur through a contact-independent manner.

Isolation of multipotent PCs from the adult OMLP offers several important advantages over the use of other ASC sources. The oral mucosa is a readily accessible biopsy site requiring minimal invasion for the patient with biopsies taken during routine dental procedures, thus offering huge advantages for the patient when compared to surgery such as bone marrow aspiration for the isolation of BMSCs. Rapid healing responses and minimal scar formation within buccal mucosa also make isolation of PCs more appealing than from the skin, which results in scar formation. The isolation of autologous progenitor cells at any age also offers the reassurance of immunocompatability. (Patients can rarely use their own bone marrow aspirate for stem cell applications, relying on allogeneic donors. These obviously come with a high risk of immune-rejection, which would be overcome with the use of autologous PCs). Additionally, our data also show the potent effect of OMLP-PCs on lymphocyte proliferation and the suppression of an allogeneically induced immune response thus showing that adult OMLP PCs also have use in allogeneic grafting and, further, in the treatment of immune related disorders such as, but not limited to, GVHD or autoimmunity.

### References

1. Rao MS and MP Mattson. (2001). Stem cells and aging: expanding the possibilities. Mech Ageing Dev 122:713-34.
2. Wright LS, KR Prowse, K Wallace, MH Linskens and CN Svendsen. (2006). Human progenitor cells isolated from the developing cortex undergo decreased neurogenesis and eventual senescence following expansion in vitro. Exp Cell Res 312:2107-20.
3. Zimmermann S, M Voss, S Kaiser, U Kapp, CF Waller and UM Martens. (2003). Lack of telomerase activity in human mesenchymal stem cells. Leukemia 17:1146-9.
4. Irwin CR, M Picardo, I Ellis, P Sloan, A Grey, M McGurk and SL Schor. (1994). Inter- and intra-site heterogeneity in the expression of fetal-like phenotypic characteristics by gingival fibroblasts: potential significance for wound healing. J Cell Sci 107 (Pt 5):1333-46.
5. Schor SL, I Ellis, CR Irwin, J Banyard, K Seneviratne, C Dolman, AD Gilbert and DM Chisholm. (1996). Subpopulations of fetal-like gingival fibroblasts: characterisation and potential significance for wound healing and the progression of periodontal disease. Oral Dis 2:155-66.
6. Stephens P, KJ Davies, T al-Khateeb, JP Shepherd and DW Thomas. (1996). A comparison of the ability of intra-oral and extra-oral fibroblasts to stimulate extracellular matrix reorganization in a model of wound contraction. J Dent Res 75:1358-64.
7. Stephens P, KJ Davies, N Occleston, RD Pleass, C Kon, J Daniels, PT Khaw and DW Thomas. (2001). Skin and oral fibroblasts exhibit phenotypic differences in extracellular matrix reorganization and matrix metalloproteinase activity. Br J Dermatol 144:229-37.
8. Stephens P, S Hiscox, H Cook, WG Jiang, W Zhiquiang and DW Thomas. (2001). Phenotypic variation in the production of bioactive hepatocyte growth factor/scatter factor by oral mucosal and skin fibroblasts. Wound Repair Regen 9:34-43.
9. Clausen H, P Vedtofte, D Moe, E Dabelsteen, TT Sun and B Dale. (1986). Differentiation-dependent expression of keratins in human oral epithelia. J Invest Dermatol 86:249-54.
10. Jones PH and FM Watt. (1993). Separation of human epidermal stem cells from transit amplifying cells on the basis of differences in integrin function and expression. Cell 73:713-24.
11. Presland RB and BA Dale. (2000). Epithelial structural proteins of the skin and oral cavity: function in health and disease. Crit Rev Oral Biol Med 11:383-408.
12. Stephens P and P Genever. (2006). Non-epithelial oral mucosal progenitor cell populations. Oral Diseases.
13. Wada N, D Menicanin, S Shi, PM Bartold and S Gronthos. (2009). Immunomodulatory properties of human periodontal ligament stem cells. J Cell Physiol 219:667-76.
14. Ringden O, M Uzunel, I Rasmusson, M Remberger, B Sundberg, H Lonnies, HU Marschall, A Dlugosz, A Szakos, Z Hassan, B Omazic, J Aschan, L Barkholt and K Le Blanc. (2006). Mesenchymal stem cells for treatment of therapy-resistant graft-versus-host disease. Transplantation 81:1390-7.
15. Dowthwaite GP, JC Bishop, SN Redman, IM Khan, P Rooney, DJ Evans, L Haughton, Z Bayram, S Boyer, B Thomson, MS Wolfe and CW Archer. (2004). The surface of articular cartilage contains a progenitor cell population. J Cell Sci 117:889-97.
16. Fernandes KJ, IA McKenzie, P Mill, KM Smith, M Akhavan, F Barnabe-Heider, J Biernaskie, A Junek, NR Kobayashi, JG Toma, DR Kaplan, PA Labosky, V Rafuse, CC Hui and FD Miller. (2004). A dermal niche for multipotent adult skin-derived precursor cells. Nat Cell Biol 6:1082-93.
17. Yu H, D Fang, SM Kumar, L Li, TK Nguyen, G Acs, M Herlyn and X Xu. (2006). Isolation of a novel population of multipotent adult stem cells from human hair follicles. Am J Pathol 168:1879-88.
18. Toma JG, IA McKenzie, D Bagli and FD Miller. (2005). Isolation and characterization of multipotent skin-derived precursors from human skin. Stem Cells 23:727-37.
19. Cristofalo VJ, RG Allen, RJ Pignolo, BG Martin and JC Beck. (1998). Relationship between donor age and the replicative lifespan of human cells in culture: a reevaluation. Proc Natl Acad Sci U S A 95:10614-9.
20. Henderson JK, JS Draper, HS Baillie, S Fishel, JA Thomson, H Moore and PW Andrews. (2002). Preimplantation human embryos and embryonic stem cells show comparable expression of stage-specific embryonic antigens. Stem Cells 20:329-37.
21. Nakahara H, SP Bruder, SE Haynesworth, JJ Holecek, MA Baber, VM Goldberg and AI Caplan. (1990). Bone and cartilage formation in diffusion chambers by subcultured cells derived from the periosteum. Bone 11:181-8.
22. Wege H, MS Chui, HT Le, JM Tran and MA Zern. (2003). SYBR Green real-time telomeric repeat amplification protocol for the rapid quantification of telomerase activity. Nucleic Acids Res 31 :E3-3.
23. Kim NW and F Wu. (1997). Advances in quantification and characterization of telomerase activity by the telomeric repeat amplification protocol (TRAP). Nucleic Acids Res 25:2595-7.
24. Li Y, S Powell, E Brunette, J Lebkowski and R Mandalam. (2005). Expansion of human embryonic stem cells in defined serum-free medium devoid of animal-derived products. Biotechnol Bioeng 91:688-98.
25. Rasmusson I, O Ringden, B Sundberg and K Le Blanc. (2005). Mesenchymal stem cells inhibit lymphocyte proliferation by mitogens and alloantigens by different mechanisms. Exp Cell Res 305:33-41.
26. Kotobuki N, M Hirose, H Funaoka and H Ohgushi. (2004). Enhancement of in vitro osteoblastic potential after selective sorting of osteoblasts with high alkaline phosphatase activity from human osteoblast-like cells. Cell Transplant 13:377-83.
27. Dominici M, K Le Blanc, I Mueller, I Slaper-Cortenbach, F Marini, D Krause, R Deans, A Keating, D Prockop and E Horwitz. (2006). Minimal criteria for defining multipotent mesenchymal stromal cells. The International Society for Cellular Therapy position statement. Cytotherapy 8:315-7.
28. Gotherstrom C, O Ringden, C Tammik, E Zetterberg, M Westgren and K Le Blanc. (2004). Immunologic properties of human fetal mesenchymal stem cells. Am J Obstet Gynecol 190:239-45.
29. Le Blanc K, C Tammik, K Rosendahl, E Zetterberg and O Ringden. (2003). HLA expression and immunologic properties of differentiated and undifferentiated mesenchymal stem cells. Exp Hematol 31:890-6.

### SEQUENCE LISTING

<110> University College Cardiff Consultants Ltd
   STEPHENS, Philip
   DAVIES, Lindsay K
<120> Novel Adult Progenitor Cell
<130> UC3.39
<140> GB 0820012.3
   <141> 2008-10-31
<150> GB 0810841.7
   <151> 2008-06-13
<150> GB 0811865.5
   < 151 > 2008-06-27
<160> 28
<170> Patentln version 3.1
<210> 1
   <211> 20
   <212> DNA
   <213> Human
<400> 1
   tgcttattca ggacagccct 20
<210> 2
   <211> 22
   <212> DNA
   <213> Human
<400> 2
   tctggtcttc tgtttcttga ct 22
<210> 3
   <211> 20
   <212> DNA
   <213> Human
<400> 3
   cgaccatctg ccgctttgag 20
<210> 4
   <211> 20
   <212> DNA
   <213> Human
<400> 4
   ccccctgtcc cccattccta 20
<210> 5
   <211> 20
   <212> DNA
   <213> Human
<400> 5
   cggaagagtg tctggagcaa 20
<210> 6
   <211> 19
   <212> DNA
   <213> Human
<400> 6
   ggatgaagcg gagtctgga 19
<210> 7
   <211> 20
   <212> DNA
   <213> Human
<400> 7
   ctacctgtca gacgtggcct 20
<210> 8
   <211> 22
   <212> DNA
   <213> Human
<400> 8
   cgcagagggt tgtattggtt cg 22
<210> 9
   <211> 20
   <212> DNA
   <213> Human
<400> 9
   aagcagagtc ccagtgccta 20
<210> 10
   <211> 20
   <212> DNA
   <213> Human
<400> 10
   cagggggcac tgacagtaat 20
<210> 11
   <211> 21
   <212> DNA
   <213> Human
<400> 11
   cagtcgcctg agaatgatca c 21
<210> 12
   <211> 21
   <212> DNA
   <213> Human
<400> 12
   gaatgaccag cagcaagaca g 21
<210> 13
   <211> 20
   <212> DNA
   <213> Human
<400> 13
   atccttgtcc tcatgctgct 20
<210> 14
   <211> 20
   <212> DNA
   <213> Human
<400> 14
   gccttgaagc cattcttgtc 20
<210> 15
   <211> 20
   <212> DNA
   <213> Human
<400> 15
   ctacaatggt ggcatctgtg 20
<210> 16
   <211> 20
   <212> DNA
   <213> Human
<400> 16
   gcgatacccg ttgatctcat 20
<210> 17
<211> 18
   <212> DNA
   <213> Human
<400> 17
   aatccgtcga gcagagtt 18
<210> 18
   <211> 24
   <212> DNA
   <213> Human
<400> 18
   gcggcgctta cccttaccct aacc 24
<210> 19
   <211> 20
   <212> DNA
   <213> Human
<400> 19
   aaccccaaga tgcacaactc 20
<210> 20
   <211> 20
   <212> DNA
   <213> Human
<400> 20
   cggggccggt atttataatc 20
<210> 21
   <211> 20
   <212> DNA
   <213> Human
<400> 21
   aceeacacag gtgagaaacc 20
<210> 22
   <211> 20
   <212> DNA
   <213> Human
<400> 22
   atgtgtaagg cgaggtggtc 20
<210> 23
   <211> 20
   <212> DNA
   <213> Human
<400> 23
   cctgctcgtg ctgactctgg 20
<210> 24
   <211> 21
   <212> DNA
   <213> Human
<400> 24
   catcctgtcc caccagtttg g 21
<210> 25
   <211> 20
   <212> DNA
   <213> Human
<400> 25
   ccggcctctt cccttaccag 20
<210> 26
   <211> 20
   <212> DNA
   <213> Human
<400> 26
   ccaccgactt cttggccttg 20
<210> 27
   <211> 20
   <212> DNA
   <213> Human
<400> 27
   ccacaggccg agaaggagaa 20
<210> 28
   <211> 20
   <212> DNA
   <213> Human
<400> 28
   ccagcagccc tgaaagatgc 20

### Organization Applicant

Street: 30-36 Cardiff Road
City: Cardiff
State:
Country: United Kingdom
PostaICode : CF24 0DE
PhoneNumber:
FaxNumber:
EmailAddress :
   <110> OrganizationName : University College Cardiff Consultants Ltd

### Individual Applicant

Street: University College Cardiff Consultants Ltd, 30-36 Cardiff Road
City: Cardiff
State:
Country: United Kingdom
PostaICode : CF24 0DE
PhoneNumber :
FaxNumber:
EmailAddress :
   <110> LastName : STEPHENS
   <110> FirstName : Philip
   <110> Middlelnitial :
   <110> Suffix :

### Individual Applicant

Street: University College Cardiff Consultants Ltd, 30-36 Cardiff Road
City: Cardiff
State :
Country: United Kingdom
PostaICode : CF24 0DE
PhoneNumber:
FaxNumber :
EmailAddress :
   <110> LastName : DAVIES
   <110> FirstName : Lindsay
   <110> Middlelnitial : K
   <110> Suffix:

### Application Project

<120> Title : Novel Adult Progenitor Cell
<130> AppFileReference : UC3.39
<140> CurrentAppNumber: GB 0820012.3
<141> CurrentFilingDate : 2008-10-31

### Earlier Applications

<150> PriorAppNumber : GB 0810841.7
<151> PriorFilingDate : 2008-06-13

### Earlier Applications

<150> PriorAppNumber: GB 0811865.5
<151> PriorFilingDate : 2008-06-27

### Sequence

<213> OrganismName : Human
<400> PreSequenceString :
   tgcttattca ggacagccct 20
<212> Type : DNA
<211> Length: 20
   SequenceName : UC3.39 - SEQ ID 1
   SequenceDescription :

### Sequence

<213> OrganismName : Human
<400> PreSequenceString :
   tctggtcttc tgtttcttga ct 22
<212> Type : DNA
<211> Length: 22
   SequenceName : UC3.39 - SEQ ID 2
   SequenceDescription :

### Sequence

<213> OrganismName : Human
<400> PreSequenceString :
   cgaccatctg ccgctttgag 20
<212> Type: DNA
<211> Length: 20
   SequenceName : UC3.39 - SEQ ID 3
   SequenceDescription :

### Sequence

<213> OrganismName : Human
<400> PreSequenceString :
   ccccctgtcc cccattccta 20
<212> Type : DNA
<211> Length: 20
   SequenceName : UC3.39 - SEQ ID 4
   SequenceDescription :

### Sequence

<213> OrganismName: Human
<400> PreSequenceString :
   cggaagagtg tctggagcaa 20
<212> Type : DNA
<211> Length : 20
   SequenceName : UC3.39 - SEQ ID 5
   SequenceDescription:

### Sequence

<213> OrganismName : Human
<400> PreSequenceString :
   ggatgaagcg gagtctgga 19
<212> Type : DNA
<211> Length: 19
   SequenceName : UC3.39 - SEQ ID 6
   SequenceDescription :

### Sequence

<213> OrganismName: Human
<400> PreSequenceString :
   ctacctgtca gacgtggcct 20
<212> Type : DNA
<211> Length : 20
   SequenceName : UC3.39 - SEQ ID 7
   SequenceDescription :

### Sequence

<213> OrganismName: Human
<400> PreSequenceString :
   cgcagagggt tgtattggtt cg 22
<212> Type: DNA
<211> Length: 22
   SequenceName : UC3.39 - SEQ ID 8
   SequenceDescription :

### Sequence

<213> OrganismName : Human
<400> PreSequenceString :
   aagcagagtc ccagtgccta 20
<212> Type: DNA
<211>Length : 20
   SequenceName : UC3.39 - SEQ ID 9
   SequenceDescription :

### Sequence

<213> OrganismName: Human
<400> PreSequenceString :
   cagggggcac tgacagtaat 20
<212> Type : DNA
<211> Length: 20
   SequenceName : UC3.39 - SEQ ID 10
   SequenceDescription :

### Sequence

<213> OrganismName : Human
<400> PreSequenceString :
   cagtcgcctg agaatgatca c 21
<212> Type : DNA
<211> Length : 21
   SequenceName : UC3.39 - SEQ ID 11
   SequenceDescription :

### Sequence

<213> OrganismName: Human
<400> PreSequenceString :
   gaatgaccag cagcaagaca g 21
<212> Type : DNA
<211> Length: 21
   SequenceName : UC3.39 - SEQ ID 12
   SequenceDescription :

### Sequence

<213> OrganismName : Human
<400> PreSequenceString :
   atccttgtcc tcatgctgct 20
<212> Type : DNA
<211> Length : 20
   SequenceName : UC3.39 - SEQ ID 13
   SequenceDescription :

### Sequence

<213> OrganismName: Human
<400> PreSequenceString :
   gccttgaagc cattcttgtc 20
<212> Type : DNA
<211> Length: 20
   SequenceName : UC3.39 - SEQ ID 14
   SequenceDescription :

### Sequence

<213>OrganismName : Human
<400> PreSequenceString :
   ctacaatggt ggcatctgtg 20
<212> Type : DNA
<211> Length : 20
   SequenceName : UC3.39 - SEQ ID 15
   SequenceDescription :

### Sequence

<213> OrganismName: Human
<400> PreSequenceString :
   gcgatacccg ttgatctcat 20
<212> Type : DNA
<211> Length : 20
   SequenceName : UC3.39 - SEQ ID 16
   SequenceDescription :

### Sequence

<213> OrganismName: Human
<400> PreSequenceString :
   aatccgtcga gcagagtt 18
<212> Type: DNA
<211> Length: 18
   SequenceName : UC3.39 - SEQ ID 17
   SequenceDescription :

### Sequence

<213> OrganismName : Human
<400> PreSequenceString :
   gcggcgctta cccttaccct aacc 24
<212> Type: DNA
<211> Length: 24
   SequenceName : UC3.39 - SEQ ID 18
   SequenceDescription :

### Sequence

<213> OrganismName : Human
<400> PreSequenceString :
   aaccccaaga tgcacaactc 20
<212> Type : DNA
<211> Length: 20
   SequenceName : UC3.39 - SEQ ID 19
   SequenceDescription :

### Sequence

<213> OrganismName : Human
<400> PreSequenceString :
   cggggccggt atttataatc 20
<212> Type : DNA
<211> Length: 20
   SequenceName : UC3.39 - SEQ ID 20
   SequenceDescription:

### Sequence

<213> OrganismName : Human
<400> PreSequenceSfring :
   acccacacag gtgagaaacc 20
<212> Type: DNA
<211> Length : 20
   SequenceName : UC3.39 - SEQ ID 21
   SequenceDescription :

### Sequence

<213> OrganismName : Human
<400> PreSequenceString :
   atgtgtaagg cgaggtggtc 20
<212> Type: DNA
<211> Length: 20
   SequenceName : UC3.39 - SEQ ID 22
   SequenceDescription :

### Sequence

<213> OrganismName : Human
<400> PreSequenceString:
   cctgctcgtg ctgactctgg 20
<212> Type : DNA
<211> Length: 20
   SequenceName : UC3.39 - SEQ ID 23
   Sequence Description :

### Sequence

<213> OrganismName: Human
<400> PreSequenceString :
   catcctgtcc caccagtttg g 21
<212> Type : DNA
<211> Length: 21
   SequenceName : UC3.39 - SEQ ID 24
   SequenceDescription :

### Sequence

<213> OrganismName : Human
<400> PreSequenceString :
   ccggcctctt cccttaccag 20
<212> Type: DNA
<211> Length: 20
   SequenceName : UC3.39 - SEQ ID 25
   SequenceDescription :

### Sequence

<213> OrganismName : Human
<400> PreSequenceString :
   ccaccgactt cttggccttg 20
<212> Type : DNA
<211> Length: 20
   SequenceName : UC3.39 - SEQ ID 26
   SequenceDescription :

### Sequence

<213> OrganismName: Human
<400> PreSequenceString :
   ccacaggccg agaaggagaa 20
<212> Type : DNA
<211> Length: 20
   SequenceName : UC3.39 - SEQ ID 27
   SequenceDescription:

### Sequence

<213> OrganismName: Human
<400> PreSequenceString :
   ccagcagccc tgaaagatgc 20
<212> Type: DNA
<211> Length : 20
   SequenceName : UC3.39 - SEQ ID 28
   SequenceDescription :

## Claims

1. A method for obtaining progenitor cells (PC) from the lamina propria of the oral mucosa comprising:
a) treating a sample from the oral mucosa of a subject by removal of fatty tissue and the epithelial layer of the cells;
b) removing the remaining lamina propria extracellular matrix to generate a mixed suspension of cells;
c) isolating potential progenitor cells and allowing them to form colonies; and
d) selecting colonies with each one of the following markers characteristic of said progenitor cells: hTERT, Nanog, Oct 4, Sox2, KLF-4, Sox10, Slug, Snail and Twist.

2. A method according to claim 1 wherein step (a) involves removing fatty tissue from the oral mucosa and enzymatically digesting the tissue so as to remove the epithelial layer of the oral mucosa.

3. A method according to claims 1 or 2 wherein step (b) involves enzymatic digestion.

4. A method according to any preceding claim wherein step (c) involves differential adhesion to a cell binding agent and the subsequent culturing of binding agent-bound cells.

5. A method according to any preceding claim wherein step (c) involves either production of a clonal population or a heterogeneous population of cells.

6. A method according to any preceding claim wherein step (d) involves further assaying the cells in order to determine whether one or more of the following markers are expressed CD90, CD105, CD166, CD44, CD146, CD34, CD45, Notch1/2/or 3, Delta 1 and Jagged 2.

7. A method according to claim 6 wherein said markers are identified using fluorescence-activated cell sorting (FACS) analysis.

8. An isolated human adult progenitor cell, wherein said cell is derived from the lamina propria of the oral mucosa and further wherein said cell is multipotent, self-renewing, immunomodulatory and expresses each one of the following markers characteristic of said progenitor cells: hTERT, Nanog, Oct4, Sox2, KLF-4, Sox10, Slug, Snail and Twist.

9. An isolated human adult progenitor cell, , according to claim 8 wherein said cell is further positive for any one or more of the following markers CD90, CD105, CD166, CD44, CD146, Notch1/2/or 3, Delta 1 and Jagged 2.

10. An isolated human adult progenitor cell according to claims 8-9 wherein said cell is further negative for the cell markers CD34 and CD45.

11. Progenitor cells of claims 8-10, for use as a medicament.

12. Progenitor cells for use as a medicament according to claim 11 **characterised in that**: said cells are used to treat any one or more of the following: wounds, burns, tissue damage, inflammation, an immune disorder including autoimmunity, GVHD, allogeneic haematopoietic stem cell (HSC) transplantation or diabetes.

## Patentansprüche

1. Verfahren zum Erlangen von Stammzellen (PC) von der Lamina propria der Mundschleimhaut, umfassend:
a) Behandeln einer Probe von der Mundschleimhaut eines Patienten durch Entfernen von Fettgewebe und des Epithels der Zellen;
b) Entfernen der verbleibenden extrazellulären Matrix der Lamina propria, um eine gemischte Suspension von Zellen zu erzeugen;
c) Isolieren potenzieller Stammzellen und Zulassen, dass diese Kolonien bilden; und
d) Auswählen von Kolonien mit jedem der folgenden Marker, die für die Stammzellen charakteristisch sind: hTERT, Nanog, Oct 4, Sox2, KLF-4, Sox10, Slug, Snail und Twist.

2. Verfahren nach Anspruch 1, wobei Schritt (a) das Entfernen von Fettgewebe von der Mundschleimhaut und enzymatisches Verdauen des Gewebes einschließt, um das Epithel der Mundschleimhaut zu entfernen.

3. Verfahren nach den Ansprüchen 1 oder 2, wobei Schritt (b) enzymatisches Verdauen einschließt.

4. Verfahren nach einem der vorangehenden Ansprüche, wobei Schritt (c) differenzielle Adhäsion an einem Zellbindungsmittel und das anschließende Kultivieren von an Bindemittel gebundenen Zellen einschließt.

5. Verfahren nach einem der vorangehenden Ansprüche, wobei Schritt (c) entweder die Produktion einer klonalen Population oder einer heterogenen Population von Zellen einschließt.

6. Verfahren nach einem der vorangehenden Ansprüche, wobei Schritt (d) ferner das Testen von Zellen einschließt, um zu bestimmen, ob einer oder mehrere der folgenden Marker exprimiert werden: CD90, CD105, CD166, CD44, CD146, CD34, CD45, Notch1/2/oder 3, Delta 1 und Jagged 2.

7. Verfahren nach Anspruch 6, wobei die Marker mittels fluoreszenzaktivierter Zellsortierungs(FACS)-Analyse identifiziert werden.

8. Isolierte humane adulte Stammzelle, wobei die Zelle von der Lamina propria der Mundschleimhaut gewonnen wird und wobei die Zelle ferner multipotent, selbsterneuernd und immunmodulatorisch ist und jeden der folgenden Marker exprimiert, die für die Stammzellen charakteristisch sind: hTERT, Nanog, Oct 4, Sox2, KLF-4, Sox10, Slug, Snail und Twist.

9. Isolierte humane adulte Stammzelle nach Anspruch 8, wobei die Zelle ferner für einen oder mehrere der folgenden Marker positiv ist: CD90, CD105, CD166, CD44, CD146, Notch1/2/oder 3, Delta 1 und Jagged 2.

10. Isolierte humane adulte Stammzelle nach den Ansprüchen 8 - 9, wobei die Zelle ferner für die Zellmarker CD34 und CD45 negativ ist.

11. Stammzellen nach den Ansprüchen 8 - 10 zur Verwendung als ein Medikament.

12. Stammzellen zur Verwendung als ein Medikament nach Anspruch 11, **dadurch gekennzeichnet, dass**: die Zellen benutzt werden, um eins oder mehrere der folgenden zu behandeln: Wunden, Verbrennungen, Gewebeschäden, Entzündung, eine Immunstörung einschließlich Autoimmunität, GVHD, allogene hämatopoetische Stammzellen(HSC)-Transplantation oder Diabetes.

## Revendications

1. Procédé d'obtention de cellules progénitrices (CP) de la lamina propria de la muqueuse orale comprenant :
a) le traitement d'un échantillon de la muqueuse orale d'un sujet par l'enlèvement du tissu gras et de la couche épithéliale des cellules ;
b) l'enlèvement de la matrice extracellulaire de la lamina propria restante pour produire une suspension mixte de cellules ;
c) l'isolement de cellules progénitrices potentielles et les laisser former des colonies ; et
d) la sélection de colonies avec chacun des marqueurs suivants caractéristiques desdites cellules progénitrices : hTERT, Nanog, Oct 4, Sox2, KLF-4, Sox10, Slug, Snail et Twist.

2. Procédé selon la revendication 1, dans lequel l'étape (a) implique l'enlèvement du tissu gras de la muqueuse orale et la digestion enzymatique du tissu de manière à enlever la couche épithéliale de la muqueuse orale.

3. Procédé selon la revendication 1 ou 2 dans lequel l'étape (b) implique une digestion enzymatique.

4. Procédé selon l'une quelconque des revendications précédentes dans lequel l'étape (c) implique l'adhérence différentielle à un agent de liaison cellulaire et la culture subséquente des cellules liées à l'agent de liaison.

5. Procédé selon l'une quelconque des revendications précédentes dans lequel l'étape (c) implique la production d'une population clonale ou d'une population hétérogène de cellules.

6. Procédé selon l'une quelconque des revendications précédentes dans lequel l'étape (d) implique en outre de tester les cellules pour déterminer si un ou plusieurs des marqueurs suivants sont exprimés CD90, CD105, CD166, CD44, CD146, CD34, CD45, Notch1/2/ou 3, Delta 1 et Jagged 2.

7. Procédé selon la revendication 6 dans lequel lesdits marqueurs sont identifiés en utilisant une analyse de tri de cellules activées par fluorescence (FACS).

8. Cellule progénitrice adulte humaine isolée, dans laquelle ladite cellule est dérivée de la lamina propria de la muqueuse orale et en outre dans laquelle ladite cellule est multipotente, auto-renouvelable, immunomodulatoire et exprime chacun des marqueurs suivants caractéristiques desdites cellules progénitrices : hTERT, Nanog, Oct4, Sox2, KLF-4, Sox10, Slug, Snail et Twist.

9. Cellule progénitrice adulte humaine isolée, selon la revendication 8 dans laquelle ladite cellule est en outre positive pour l'un quelconque ou plusieurs des marqueurs suivants CD90, CD105, CD166, CD44, CD146, Notch1/2/ou 3, Delta 1 et Jagged 2.

10. Cellule progénitrice adulte humaine isolée selon les revendications 8 à 9 dans laquelle ladite cellule est en outre négative pour les marqueurs cellulaires CD34 et CD45.

11. Cellule progénitrice selon les revendications 8 à 10, pour une utilisation comme médicament.

12. Cellules progénitrices pour une utilisation comme médicament selon la revendication 11 **caractérisées en ce que** : lesdites cellules sont utilisées pour traiter l'un quelconque ou plus de ce qui suit : les blessures, les brûlures, les lésions tissulaires, l'inflammation, un trouble immunitaire comprenant l'autoimmunité, le GVHD (réaction du greffon contre l'hôte), la greffe allogènique de cellules souches hématopoïétiques (CSH) ou le diabète.
